# EUROPEAN PATENT APPLICATION

(11) **EP 1 921 089 A1**
(43) Date of publication of application: **14.05.2008**
(21) Application number: 06782236.1
(22) Date of filing: 03.08.2006
(51) Int. Cl.: C07K 14/075, A61K 38/00, A61K 39/00, A61K 48/00, A61P 31/12, C12N 5/06, C12N 15/09, C12Q 1/06, G01N 33/50

(54) **CYTOTOXIC T-CELL EPITOPE PEPTIDE AND USE THEREOF**

(30) Priority: 03.08.2005 JP 2005226033
(71) Applicant: Medical and Biological Laboratories Co., Ltd., Nagoya-shi, Aichi 460-0002 (JP)
(72) Inventor: TOJI, Shingo, MEDICAL & BIOLOGICAL LAB. CO. INC., Ina-shi, Nagano 396-0002 (JP); KIM, Yool-Ja, MEDICAL & BIOLOGICAL LAB. CO. INC., Ina-shi, Nagano 396-0002 (JP); SUZUKI, Susumu, MEDICAL & BIOLOGICAL LAB. CO. INC., Ina-shi, Nagano 396-0002 (JP)
(74) Representative: Denison, Christopher Marcus
(86) International application number: PCT/JP2006/315376
(87) International publication number: WO 2007/015540

(57) **Abstract**

The successful identification of epitope peptides specific to adenovirus belonging to subgroup B and epitope peptides exhibiting specificity to all adenoviruses using hexon proteins which exhibit the highest homology among genes of adenoviruses of various subgroups is herein described. The peptides have a function capable of efficiently inducing adenovirus-specific cytotoxic T cells (CTLs). Thus, the peptides disclosed herein find utility as vaccines for active immunization. Furthermore, CTLs induced by such peptides find utility as passive immunotherapeutic agents.

SEQUENCE LISTING

<110>  MEDICAL AND BIOLOGICAL LABORATORIES CO., LTD.

<120>  CYTOTOXIC T-CELL EPITOPE PEPTIDE AND USE THEREOF

<130>  M3-A0501P

<150>  JP 2005-226033
<151>  2005-08-03

<160>  26
<170>  PatentIn version 3.3

<210>  1
<211>  9
<212>  PRT
<213>  Artificial

<220>
<223>  An artificially synthesized epitope peptide sequence

<400>  1
Asp Tyr Leu Ser Ala Ala Asn Met Leu
1               5

<210>  2
<211>  9
<212>  PRT
<213>  Artificial
<220>
<223>  An artificially synthesized epitope peptide sequence

<400>  2
Leu Tyr Ala Asn Ser Ala His Ala Leu

1               5

<210>  3
<211>  9
<212>  PRT
<213>  Artificial
<220>
<223>  An artificially synthesized epitope peptide sequence

<400>  3
Val Tyr Ser Gly Ser Ile Pro Tyr Leu
1               5

<210>  4
<211>  9
<212>  PRT
<213>  Artificial

<220>
<223>  An artificially synthesized epitope peptide sequence

<400>  4
Thr Tyr Phe Asn Leu Gly Asn Lys Phe
1               5

<210>  5
<211>  9
<212>  PRT
<213>  Artificial

<220>
<223>  An artificially synthesized epitope peptide sequence

<400> 5
Leu Tyr Ser Asn Val Ala Leu Tyr Leu
1               5

<210> 6
<211> 9
<212> PRT
<213> Artificial

<220>
<223> An artificially synthesized epitope peptide sequence

<400> 6
Gly Tyr Lys Asp Arg Met Tyr Ser Phe
1               5

<210> 7
<211> 9
<212> PRT
<213> Artificial

<220>
<223> An artificially synthesized epitope peptide sequence

<400> 7
Lys Tyr Thr Pro Ser Asn Val Thr Leu
1               5

<210> 8
<211> 9
<212> PRT
<213> Artificial

<220>
<223>  An artificially synthesized epitope peptide sequence

<400>  8
Ser Tyr Gln Leu Leu Leu Asp Ser Leu
1               5

<210>  9
<211>  9
<212>  PRT
<213>  Artificial

<220>
<223>  An artificially synthesized epitope peptide sequence

<400>  9
Asn Tyr Asn Ile Gly Tyr Gln Gly Phe
1               5

<210>  10
<211>  9
<212>  PRT
<213>  Artificial

<220>
<223>  An artificially synthesized epitope peptide sequence

<400>  10
Asn Tyr Ile Gly Phe Arg Asp Asn Phe
1               5

<210>  11
<211>  9

<212>  PRT
<213>  Artificial

<220>
<223>  An artificially synthesized epitope peptide sequence

<400>  11
Thr Tyr Pro Val Leu Glu Glu Met Phe
1               5


<210>  12
<211>  9
<212>  PRT
<213>  Artificial

<220>
<223>  An artificially synthesized epitope peptide sequence

<400>  12
Gln Tyr Asp Pro Val Ala Ala Leu Phe
1               5


<210>  13
<211>  9
<212>  PRT
<213>  Artificial

<220>
<223>  An artificially synthesized epitope peptide sequence

<400>  13
Arg Tyr Leu Arg Asp Gln Gln Leu Leu
1               5

<210>  14
<211>  9
<212>  PRT
<213>  Artificial

<220>
<223>  An artificially synthesized epitope peptide sequence

<400>  14
Thr Tyr Phe Ser Leu Asn Asn Lys Phe
1               5

<210>  15
<211>  9
<212>  PRT
<213>  Artificial

<220>
<223>  An artificially synthesized epitope peptide sequence

<400>  15
Asp Tyr Leu Cys Ala Ala Asn Met Leu
1               5

<210>  16
<211>  9
<212>  PRT
<213>  Artificial

<220>
<223>  An artificially synthesized epitope peptide sequence

<400>  16

Val Tyr Ser Gly Thr Ile Pro Tyr Leu
1               5

<210>  17
<211>  9
<212>  PRT
<213>  Artificial

<220>
<223>  An artificially synthesized epitope peptide sequence

<400>  17
Thr Tyr Ser Gly Ser Ile Pro Tyr Leu
1               5

<210>  18
<211>  9
<212>  PRT
<213>  Artificial

<220>
<223>  An artificially synthesized epitope peptide sequence

<400>  18
Thr Tyr Ser Gly Ser Val Pro Tyr Leu
1               5

<210>  19
<211>  9
<212>  PRT
<213>  Artificial

<220>

<223>  An artificially synthesized epitope peptide sequence

<400>  19
Thr Tyr Phe Thr Leu Gly Asn Lys Phe
1               5


<210>  20
<211>  9
<212>  PRT
<213>  Artificial

<220>
<223>  An artificially synthesized epitope peptide sequence

<400>  20
Thr Tyr Phe Ser Met Gly Asn Lys Phe
1               5


<210>  21
<211>  9
<212>  PRT
<213>  Artificial

<220>
<223>  An artificially synthesized epitope peptide sequence

<400>  21
Thr Tyr Phe Ser Leu Gly Asn Lys Phe
1               5


<210>  22
<211>  9
<212>  PRT

<213>  Artificial

<220>
<223>  An artificially synthesized epitope peptide sequence

<400>  22
Leu Tyr Ser Asn Val Gly Leu Tyr Leu
1               5

<210>  23
<211>  9
<212>  PRT
<213>  Artificial

<220>
<223>  An artificially synthesized epitope peptide sequence

<400>  23
Leu Tyr Ser Asn Ile Ala Leu Tyr Leu
1               5

<210>  24
<211>  9
<212>  PRT
<213>  Artificial

<220>
<223>  An artificially synthesized epitope peptide sequence

<400>  24
Leu Tyr Ala Asn Val Ala Leu Tyr Leu
1               5

<210> 25
<211> 9
<212> PRT
<213> Artificial

<220>
<223> An artificially synthesized epitope peptide sequence

<400> 25
Ser Tyr Lys Asp Arg Met Tyr Ser Phe
1               5

<210> 26
<211> 9
<212> PRT
<213> Artificial

<220>
<223> An artificially synthesized epitope peptide sequence

<400> 26
Arg Lys Lys Arg Arg Gln Arg Arg Arg
1               5

## Description

### Technical Field

The present invention relates to adenovirus-specific cytotoxic T cell epitope peptides, vaccines for treating or preventing adenoviral infection using such peptides, and passive immunotherapeutic agents against adenovirus.

### Background Art

Adenovirus is a virus known to cause respiratory infections represented by pneumonia; ophthalmic infections represented by pool fever and epidemic keratoconjunctivitis; gastrointestinal infections such as gastroenteritis; urogenital infections such as hemorrhagic cystitis and urethritis; and others. Adenovirus is known to at times cause severe symptoms in newborn infants. However, in adults, the symptoms are rarely worsened, so long as they receive adequate treatment. Adenovirus is used as a relatively safe viral vector in gene therapy, and in the research and development of vaccines. However, when patients are immunocompromised as a result of congenital immune deficiency, HIV (human immunodeficiency virus) infection, transplantation, or the like, adenovirus can cause hepatitis, pneumonia, encephalitis, or hemorrhagic cystitis, and thus, strongly impacts the patient prognosis and mortality (see Non-Patent Documents 1 to 3).

In recent years, due to the expansion and repletion of the bone-marrow and cord blood banks, hematopoietic stem cell transplantation between nonrelatives or with the use of cord blood has markedly increased. However, one month after transplantation, sepsis caused by bacteria and fungi, or stomatitis caused by herpes simplex virus (HSV) is likely to arise. About one to six months after transplantation, there are high risks of developing interstitial pneumonia and hepatitis caused by human cytomegalovirus (HCMV), hemorrhagic cystitis caused by adenovirus, herpes zoster caused by varicella-zoster virus (VZV), B-cell lymphoproliferative disorder (BLPD) caused by Epstein-Barr virus (EBV), and the like. The success of transplantation depends on the ability to control such infections. Thus, from a practical standpoint of transplantation, measures against infection are of the utmost importance. While problem can be somewhat resolved through pretreatment with antibiotics against bacteria and fungi or effective anti-viral agents against HSV, HCMV, and VZV, there remains no effective therapeutic method for anti-viral agent-resistant HCMV infection, B-cell lymphoproliferative disorder, or adenovirus infection.

Recently, so-called cellular immunotherapy for anti-viral agent-resistant HCMV infection and BLPD, a process in which immunocompetent cells that can eliminate cells infected with the causative virus are infused into patient bodies, has been applied and is achieving effective results (see Non-Patent Documents 4 to 6). However, there is still no effective therapeutic method for adenovirus infection; only symptomatic and supportive therapy is available. Donor leukocyte infusion has been attempted as therapy in some cases (see Non-Patent Documents 7 and 8).

Given the above-described medical circumstances and social backgrounds, a new method for safely and effectively controlling adenovirus is needed in the art.
[Non-Patent Document 1] Flomenberg P and three other authors, "Characterization of human proliferative T cell responses to adenovirus" J. Infect. Dis., 1995, Vol. 171, p. 1090-1096
[Non-Patent Document 2] Hale GA and six other authors, "Adenovirus infection after pediatric bone marrow transplantation" Bone Marrow Transplant, 1999, Vol. 23, p. 277-282
[Non-Patent Document 3] Howard DS and seven other authors "Adenovirus infections in hematopoietic stem cell transplant recipients" Clin. Infect. Dis., 1999, Vol. 29, p. 1494-1501
[Non-Patent Document 4] Einsele H and thirteen other authors, "Infusion of cytomegalovirus (CMV)-specific T cells for the treatment of CMV infection not responding to antiviral chemotherapy" Blood, 2002, Vol. 99, p. 3916-3922
[Non-Patent Document 5] Heslop HE and Rooney CM "Adoptive cellular immunotherapy for EBV lymphoproliferative disease" Immunol. Rev. 1997, Vol. 157, p. 217-222
[Non-Patent Document 6] Walter EA and six other authors "Reconstitution of cellular immunity against cytomegalovirus in recipients of allogeneic bone marrow by transfer of T cell clones from the donor" N. Engl. J. Med., 1995, Vol. 333, p. 1038-1044
[Non-Patent Document 7] Hromas R and four other authors "Donor leukocyte infusion as therapy of life-threatening adenoviral infections after T-cell-depleted bone marrow transplantation" Blood, 1994, Vol. 84, p. 1689-1690
[Non-Patent Document 8] Chakrabarti S and four other authors "Adenovirus infections following haematopoietic cell transplantation: is there a role for adoptive immunotherapy?" Bone Marrow Transplant, 2000, Vol. 26, p. 305-307

### Disclosure of the Invention

### [Problems to be Solved by the Invention]

The present invention was achieved in view of the above circumstances. An objective of the present invention is to provide adenovirus-specific cytotoxic T cell epitope peptides, vaccines for treating or preventing adenovirus infection using such peptides, passive immunotherapeutic agents against adenovirus, and methods for quantifying adenovirus-specific CTL.

### [Means for Solving the Problems]

The present inventors conducted dedicated studies to resolve the objective described above.

The main immunocompetent cell that controls the activity of adenovirus-infected cells is the cytotoxic T cell (CTL; hereinafter referred to as CTL). Since the CTL is capable of finding and destroying adenovirus-infected cells, effective use of its function may lead to development of new diagnostic and therapeutic methods for adenovirus-associated diseases. The techniques of the present invention were developed with this idea in mind.

Fifty-one serotypes of adenoviruses have been reported and are categorized into six subgroups (A to F). Highly pathogenic viruses are categorized into subgroups A, B, and C (Flomenberg P, Piaskowski V, Truitt RL, Casper JT., Characterization of human proliferative T cell responses to adenovirus., J Infect Dis., 171:1090-1096 (1995); Carrigan DR., Adenovirus infections in immunocompromised patients., Am J Med., 102:71-74 (1997)).

The adenoviruses that have been reported the most to cause hepatitis, pneumonia, encephalitis, hemorrhagic cystitis, or such in immunocompromised persons as a result of congenital immune deficiency, AIDS, and post-transplantation immunosuppressants and thus strongly impact the patient prognosis and mortality are categorized into subgroup B.

Epitope peptides specific to subgroup B adenoviruses as well as epitope peptides specific to all adenoviruses were successfully identified herein through the use of hexon proteins exhibiting the highest homology among adenoviral genes. In this manner, the present invention was completed.

The epitope peptides identified herein have the function of efficiently inducing adenovirus-specific CTLs. The peptides and the nucleic acids encoding such peptides therefore find utility as vaccines (active immunotherapeutic agents) for treating or preventing adenovirus infection.

Furthermore, CTLs induced by the epitope peptides of the present invention have the function of specifically destroying adenovirus-infected cells, and thus, serve as a very useful component of passive immunotherapeutic agents.

In addition, adenovirus-specific CTLs can be quantified by using the epitope peptides. For infection management purposes as well as to determine the appropriate use of anti-viral agents and immunosuppressants, it is important to know whether adenovirus-specific CTLs are present in peripheral blood of high-risk patients. Accordingly, the present invention provides methods for quantifying CTLs using the epitope peptides.

Furthermore, the epitope peptides can be suitably altered based on the amino acid sequences of the epitope peptides identified herein, so long as they retain their desirable functions (for example, the ability to induce adenovirus-specific CTLs). Amino acids of the peptides specifically disclosed herein can be appropriately and easily modified using common genetic engineering techniques. Furthermore, it is within the scope of ordinary trials for those skilled in the art to select peptides having desired functions from peptides modified from the peptides described herein or to select cells presenting such peptides. Specifically, various types of molecules (peptides and the like) that retain the ability to induce adenovirus-specific CTLs can be prepared based on the structures of peptides specifically disclosed herein. Furthermore, molecules having a stronger ability to induce CTLs or molecules capable of inducing CTLs that exhibit a higher specificity to adenovirus-infected cells can also be produced by appropriately modifying the peptides.

The present invention relates to adenovirus-specific cytotoxic T cell epitope peptides, vaccines for treating or preventing adenovirus infection using such peptides, passive immunotherapeutic agents against adenovirus, and methods for quantifying adenovirus-specific CTLs. More specifically, the present invention provides the following:
[1] an adenovirus-specific cytotoxic T cell epitope peptide;
[2] the peptide of [1], wherein the adenovirus-specific cytotoxic T cell epitope peptide comprises at least one amino acid sequence selected from the group consisting of SEQ ID NOs: 1 to 6;
[3] the peptide of [1] comprising an amino acid sequence with a substitution, deletion, insertion, and/or addition of one or more amino acids in the amino acid sequence of any one of SEQ ID NOs: 1 to 6, which has the function capable of inducing an adenovirus-specific cytotoxic T cell;
[4] the peptide of any one of [1] to [3], wherein the peptide comprises an antigen peptide restricted by HLA-A*2402, HLA-Cw*0401, or HLA-Cw*0702 molecule and has the function capable of inducing a cytotoxic T cell having a T cell receptor capable of specifically recognizing a cell that presents a complex with HLA-A*2402, HLA-Cw*0401, or HLA-Cw*0702 molecule on the cell surface;
[5] a nucleic acid encoding the peptide of any one of [1] to [4];
[6] a vaccine for treating or preventing adenovirus infection, which comprises as an active ingredient the peptide of any one of [1] to [4];
[7] a vaccine for treating or preventing adenovirus infection, which comprises as an active ingredient the nucleic acid of [5];
[8] a vaccine for treating or preventing adenovirus infection, which comprises as an active ingredient an antigen-presenting cell that presents the peptide of any one of [1] to [4] by HLA;
[9] a passive immunotherapeutic agent against adenovirus, which comprises as an active ingredient an adenovirus-specific cytotoxic T cell obtained by stimulating a peripheral blood lymphocyte with the peptide of any one of [1] to [4] or an antigen-presenting cell that presents said peptide by HLA;
[10] a passive immunotherapeutic agent against adenovirus, which comprises as an active ingredient a cytotoxic T cell that is obtained by reacting a peripheral blood lymphocyte with a major histocompatibility antigen complex and/or major histocompatibility antigen complex-tetramer prepared from the peptide of any one of [1] to [4], allowing the formation of a complex in which said major histocompatibility antigen complex and/or major histocompatibility antigen complex-tetramer are bound with a cytotoxic T cell, and isolating the cytotoxic T cell from said complex;
[11] a method for quantifying adenovirus-specific cytotoxic T cells, which comprises: stimulating peripheral blood with the peptide of any one of [1] to [4], obtaining cytotoxic T cells specific to said virus, and assaying a cytokine and/or chemokine and/or cell surface molecule produced by the cytotoxic T cells;
[12] a method for quantifying adenovirus-specific cytotoxic T cells in peripheral blood, which comprises: preparing a major histocompatibility antigen complex-tetramer from the peptide of any one of [1] to [4], and reacting the peripheral blood with the major histocompatibility antigen complex-tetramer;
[13] a method for inducing a cytotoxic T cell, which comprises inducing a cytotoxic T cell using the peptide of any one of [1] to [4];
[14] the method of [13], wherein an adenovirus-specific cytotoxic T cell is induced by contacting the peptide of any one of [1] to [4] with a peripheral blood mononuclear cell in a culture medium containing plasma;
[15] a method for producing a passive immunotherapeutic agent against adenovirus, which comprises the step of obtaining an adenovirus-specific cytotoxic T cell by stimulating a peripheral blood lymphocyte with the peptide of any one of [1] to [4] or an antigen-presenting cell that presents said peptide by HLA; and
[16] a method for producing a passive immunotherapeutic agent against adenovirus, which comprises the step of obtaining a cytotoxic T cell by reacting a peripheral blood lymphocyte with a major histocompatibility antigen complex and/or major histocompatibility antigen complex-tetramer prepared from the peptide of any one of [1] to [4], allowing the formation of a complex in which said major histocompatibility antigen complex and/or major histocompatibility antigen complex-tetramer are bound with the cytotoxic T cell, and isolating the cytotoxic T cell from said complex.

The present invention also relates to methods for treating or preventing adenovirus infection, such methods including the step of administering any one of: the peptides described herein; nucleic acids encoding such peptides; antigen-presenting cells that present such peptides by HLA; adenovirus-specific cytotoxic T cells that are obtained through the stimulation of peripheral blood lymphocytes with such peptides or antigen-presenting cells that present the peptides by HLA; and cytotoxic T cells that are obtained through the reaction of peripheral blood lymphocytes with the major histocompatibility antigen complex and/or major histocompatibility antigen complex-tetramers prepared from such peptides, allowing the formation of a complex in which the major histocompatibility antigen complex and/or major histocompatibility antigen complex-tetramers are bound with cytotoxic T cells, and isolating the cytotoxic T cells from the complex. The present invention also relates to the use of any one of: the peptides described herein; nucleic acids encoding such peptides; antigen-presenting cells that present such peptides by HLA; adenovirus-specific cytotoxic T cells that are obtained through the stimulation of peripheral blood lymphocytes with such peptides or antigen-presenting cells that present such peptides by HLA; and cytotoxic T cells that are obtained through the reaction of peripheral blood lymphocytes with the major histocompatibility antigen complex and/or major histocompatibility antigen complex-tetramers prepared from such peptides, allowing the formation of a complex in which the major histocompatibility antigen complex and/or major histocompatibility antigen complex-tetramers are bound with cytotoxic T cells, and isolating the cytotoxic T cells from the complex, in producing vaccines for treating or preventing adenovirus infection or passive immunotherapeutic agents against adenovirus (immunotherapeutic agents).

### Brief Description of the Drawings

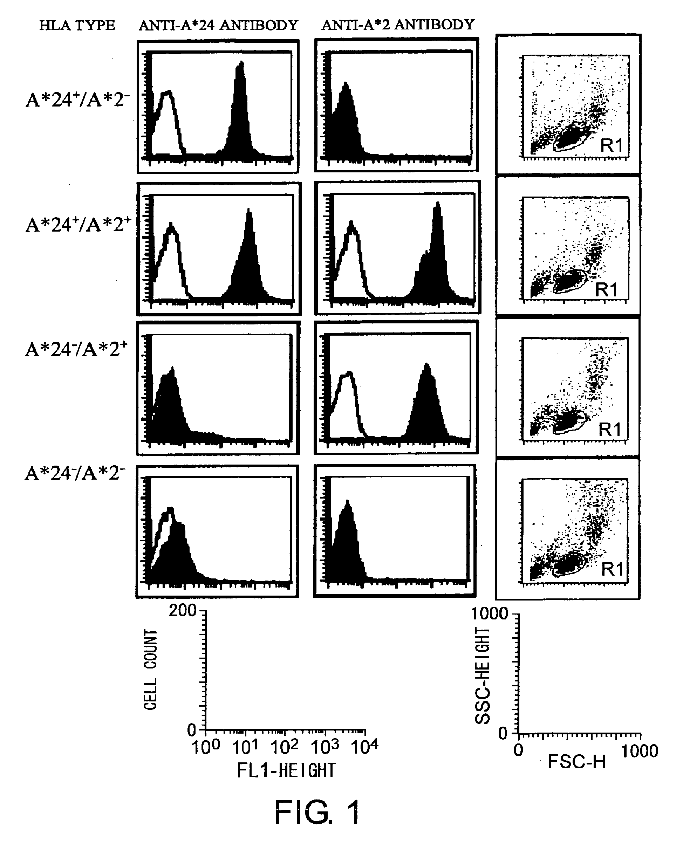

Fig. 1 is composed of diagrams that illustrate exemplary results of the HLA types determined using anti-HLA antibodies. The results of the determination of HLA types, and, as the evidence for the determination result, histograms showing the results of staining with an anti-HLA-A*24 antibody and histograms showing the results of staining with an anti-HLA-A*2 antibody are shown from the left. Furthermore, analyzed region of cells (R1) are shown in dot plots, which were developed in terms of forwardlight scattering (FSC) and sidelight scattering (SSC) indicated in the X- and Y-axes, respectively. In histograms, the X-axis indicates fluorescence intensity of FITC in the log scale, and the Y-axis indicates the cell count. The closed areas indicate the results of staining with specific antibodies; the open areas indicate the results of staining with isotype IgGs as a control. For example, HLA type A*24⁺/A*2⁻ shows the determination results obtained when the sample was reactive to the anti-HLA-A*24 antibody but not to the anti-HLA-A*2 antibody. The scales and titles of the X- and Y-axes in each histogram shown in the left two columns are separately shown at the bottom left of this figure. The scales and titles of the X- and Y-axes in each dot plot shown in the right column are separately shown at the bottom right of this figure.

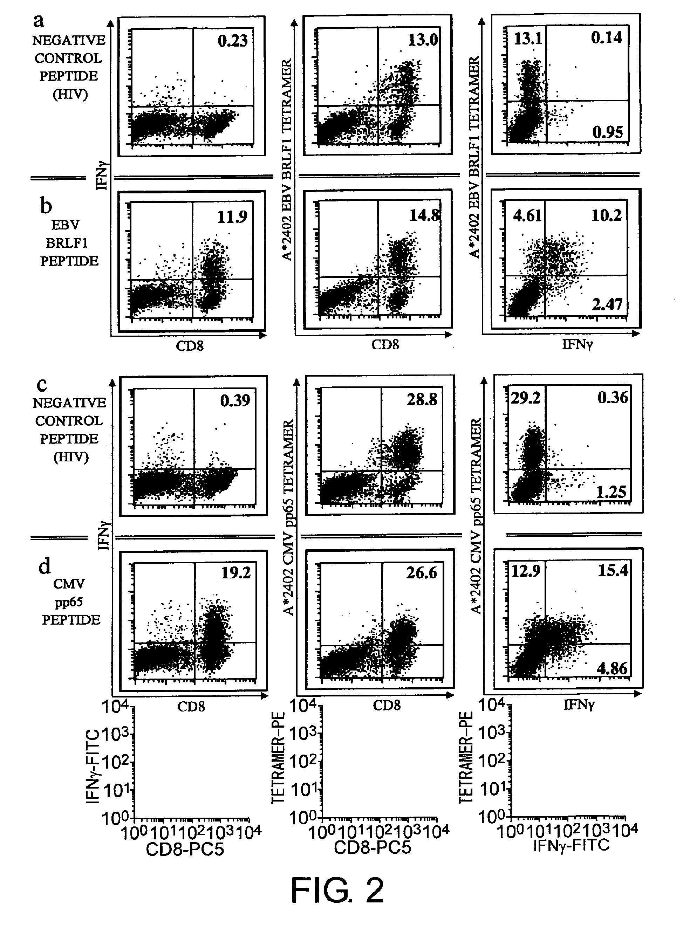

Fig. 2 is composed of diagrams that illustrate the assessment of a method for quantifying cells producing intracellular IFNγ using control peptides. This figure shows that after 13 days of stimulation with HLA-A*2402-restricted epitope peptides of EBV BRLF1 (a and b) or CMV pp65 (c and d), PBMCs from donor ID*24-2 are stimulated with negative control (HIV) peptides (a and c) and each peptide used in the stimulation (b and d), and the resulting tetramer-positive cells respond to specific peptides and produce IFNγ. The scales and titles of the X- and Y-axes in dot plots shown in each column (left, middle, or right) are separately shown at the bottom of this figure.

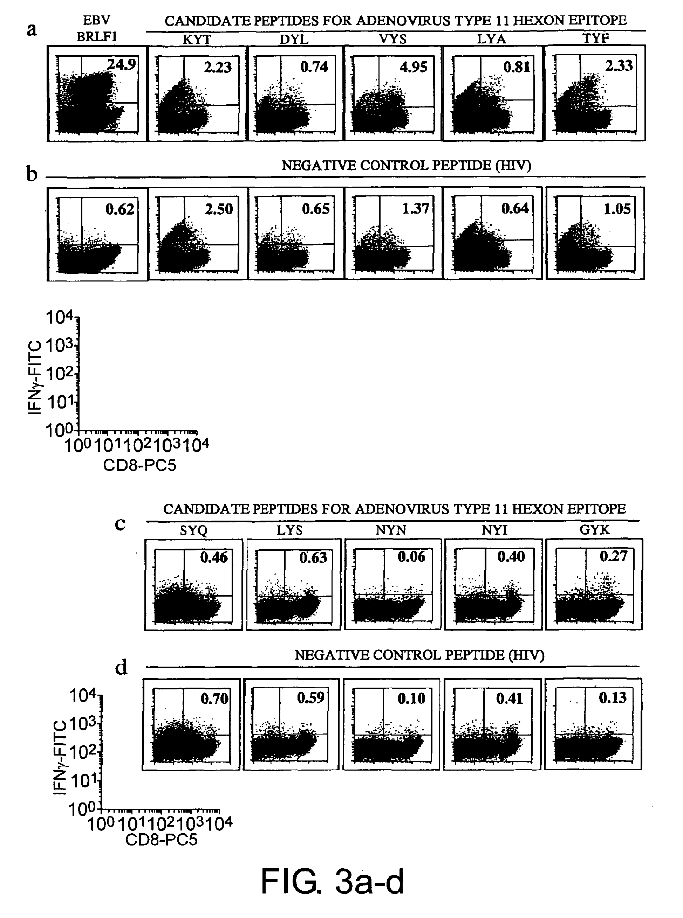

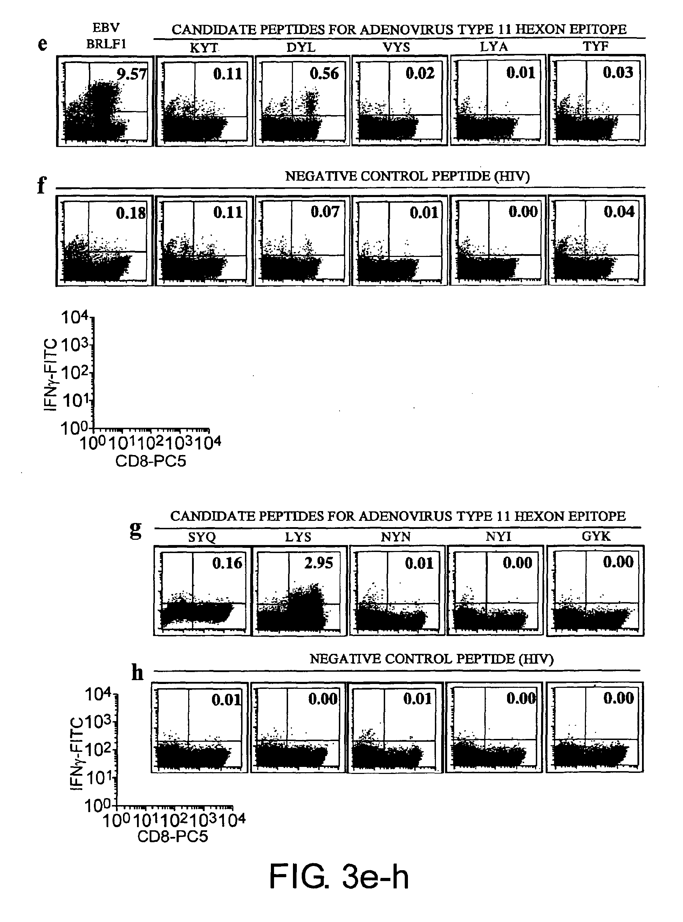

Figs. 3a-d correspond to diagrams that confirm the induction of specific CTLs (donor ID *24-8) using a method for quantifying cells producing intracellular IFNγ. The diagrams are dot plots in which the X- and Y-axes indicate the fluorescence intensities for CD8 and IFNγ in the log scale; Figures 3 a and 3c depict the results of restimulation using the same peptides as used in the induction; Figures 3b and 3d depict the results of restimulation using the negative control HIV peptides; the percentage (%) of the number of CD8-positive, IFNγ-positive cells in PBMCs is shown as a numeral in UR. The scales and titles of the X- and Y-axes of the dot plots shown in 3a and 3b are separately shown at the bottom of 3b. Likewise, the scales and titles of the X- and Y-axes of the dot plots shown in Figures 3c and 3d are separately shown at the bottom of 3d.
Figs. 3e-h correspond to diagrams that confirm the induction of specific CTLs (donor ID *24-12) using a method for quantifying cells producing intracellular IFNγ. The diagrams are dot plots in which the X- and Y-axes indicate the fluorescence intensities for CD8 and IFNγ in the log scale; Figures 3e and 3g depict the results of restimulation using the same peptides as used in the induction; Figures 3f and 3h depict the results of restimulation using the negative control HIV peptides; the percentage (%) of the number of CD8-positive, IFNγ-positive cells in PBMCs is shown as a numeral in UR. The scales and titles of the X- and Y-axes of the dot plots shown in 3e and 3f are separately shown at the bottom of 3f. Likewise, the scales and titles of the X- and Y-axes of the dot plots shown in g and h are separately shown at the bottom of 3h.
Fig. 4 is composed of a photograph and diagram that depict an example of detection of specific CTLs by ELISPOT assay.
Fig. 5 is composed of a set of diagrams that depict the results of staining using the prepared MHC-tetramer reagents. The scales and titles of the X- and Y-axes of the dot plots are separately shown at the bottom of this figure.
Fig. 6 is composed of a set of diagrams that compare the reactivity of the Negative Tetramer reagent, a negative control, to that determined by staining PBMCs from donor ID*24-14 with LYA-Tet, CD4, CD3, and CD8 after 24 days of stimulation of the PBMCs with LYA.
Fig. 7 is composed of a set of diagrams that depict the effectiveness of short-term culture to induce AdV-specific CTLs. The diagrams were obtained by staining PBMCs from donor ID*24-8 with each tetramer reagent immediately after isolation. The diagrams of Figure 7b confirm the induction of specific CTLs after 10 days of culture in the presence of a peptide, using each tetramer reagent. The scales and titles of the X- and Y-axes in each dot plot are separately shown at the bottom.
Fig. 8 is composed of a set of diagrams that assess the functionality of adenovirus-specific CTLs. Figures 8a and 8b are diagrams that assess the functionality of TYF-specific CTLs induced using PBMCs from donor ID*24-8. Figures 8c and 8d are diagrams that assess the functionality of VYS-specific CTLs induced using PBMCs from donor ID*24-2. The title of the X-axis of each dot plot is shown in the dot plot; however, the scale of the X-axis and titles and scales of the Y-axis are separately shown at the bottom of Figure 8d.
Fig. 9 is composed of a set of diagrams exemplifying the purification of CTLs using the MHC-tetramer reagents.
Fig. 10 is composed of a set of diagrams demonstrating that TYF-specific CTLs induced using PBMCs from donor ID*24-8 do not cross-react with the epitope peptide of subgroup C. The scales and titles of the X- and Y-axes of each dot plot shown at the left are separately shown at the bottom of the left dot plots; the scales and titles of the X- and Y-axes of each dot plot shown in the middle are separately shown at the bottom of the middle dot plots; and the scales and titles of the X- and Y-axes of each dot plot shown at the right are separately shown at the bottom of the right dot plots.
Fig. 11-1 is a diagram assessing the reactivity by amino acid sequence homology analysis. The homology of SEQ ID NO: 1 between subgroups is shown.
Fig. 11-2 is a diagram assessing the reactivity by amino acid sequence homology analysis. The homology of SEQ ID NO: 2 between subgroups is shown.
Fig. 11-3 is a diagram assessing the reactivity by amino acid sequence homology analysis. The homology of SEQ ID NO: 3 between subgroups is shown.
Fig. 11-4 is a diagram assessing the reactivity by amino acid sequence homology analysis. The homology of SEQ ID NO: 4 between subgroups is shown.
Fig. 11-5 is a diagram assessing the reactivity by amino acid sequence homology analysis. The homology of SEQ ID NO: 5 between subgroups is shown.
Fig. 11-6 is a diagram assessing the reactivity by amino acid sequence homology analysis. The homology of SEQ ID NO: 6 between subgroups is shown.

### Best Mode for Carrying Out the Invention

The present invention relates to T cell epitope peptides having the function capable of inducing adenovirus-specific cytotoxic T cells. Thus, the present invention provides adenovirus-specific cytotoxic T cell epitope peptides. Herein, the epitope peptides are referred to as "epitope peptides" or simply "peptides of the present invention".

The peptides of the present invention refer to biologically active linear chains of amino acid molecules that are linked together via peptide bonds between α-amino and carboxyl groups of adjacent amino acid residues. The "peptides" of the present invention are not restricted to those with a specific length and thus may be in various lengths. Thus, the peptides of the present invention also include so-called "oligopeptides" and "polypeptides".

Furthermore, such a peptide may be uncharged or in its salt form, while in some cases it can be modified by glycosylation, amidation, phosphorylation, carboxylation, phosphorylation, etc.

An example of a method for selecting candidate epitope peptides is described below.

### 1. Computer analysis

Candidates for adenovirus-specific CTL epitope peptides of the present invention can be selected through searches using multiple softwares disclosed on the Internet (Pingping Guan, Irini A. Doytchinova, Christianna Zygouri, and Darren R. Flower, MHCPred: a server for quantitative prediction of peptide-MHC binding, Nucleic Acids Res., 31:3621-3624 (2003)) which can be used to search epitope peptides consisting of 8 to 10 amino acids and containing each binding motif of a desired HLA molecule in the amino acid sequences of adenovirus proteins.

### 2. Evaluation using anchor motifs

HLA-class I molecules are primarily HLA-A, HLA-B, and HLA-C. Epitope peptides that are presented by binding with them are composed of 8 to 10 amino acids. Amino acids at the second and ninth or tenth positions from the N terminus of an epitope peptide are most critical to binding with HLA molecules, and are thus referred to the anchor motif. The anchor motif has been reported to vary depending on the type of HLA molecule. For example, the peptide best known to bind to the HLA-A2 molecule, the antigen most frequently displayed worldwide, is a peptide composed of 9 or 10 amino acid residues in which Leu is arranged at the second position and Leu or Val is arranged at the ninth or tenth position from the N terminus (T Sudo, N Kamikawaji, A Kimura, Y Date, CJ Savoie, H Nakashima, E Furuichi, S Kuhara, and T Sasazuki, Differences in MHC class I self peptide repertoires among HLA-A2 subtypes, J. Immunol., 155:4749-4756 (1995)). Alternatively, the peptide best known to bind to HLA-A24, which frequently arises in Asian populations, including the Japanese population, is a peptide composed of 9 or 10 amino acids in which Tyr, Phe, Met, or Trp is arranged at the second position and Leu, Ile, Trp, or Phe is arranged at the ninth or tenth position from the N terminus (A Kondo, J Sidney, S Southwood, MF del Guercio, E Appella, H Sakamoto, E Celis, HM Grey, RW Chesnut, and RT Kubo, Prominent roles of secondary anchor residues in peptide binding to HLA-A24 human class I molecules, J. Immunol., 155:4307-4312 (1995)). Candidates of CTL epitope peptides can be selected by searching the amino acid sequences of proteins for sequences containing such an anchor motif.

### 3. Preparation of peptide library

A library of peptides composed of about 20 amino acids in length is synthesized to cover the entire adenovirus proteins. The library is prepared so that the peptides of about 20 amino acids overlap with adjacent peptide sequences by about ten amino acids. This enables a thorough search of the entire proteins, and, once the library is prepared, the HLA restriction can also be thoroughly studied.

Not all candidate epitope peptides selected by the method described above can serve as the CTL epitope. They can be used as adenovirus-specific CTL epitopes only after the studies described below. Methods for studying the epitope peptides are described below.

### (1) Determination of epitope peptide - Method 1

Peripheral blood mononuclear cells (PBMCs) isolated from a person with a history of adenovirus infection or T cells isolated from PBMCs are suspended at a cell density of 0.1 to 2 x 10⁶ cells/ml in an adequate culture medium. Isolated and cultured adenovirus-infected cells derived from the same person are then added at 1 x 10⁵ cells/ml to the suspension. The cells are cultured in a 5% carbon dioxide gas (CO₂) incubator at 37°C for seven days. After seven days of culture, adenovirus-infected cells and interleukin 2 (IL-2) are added to the suspension. Then, the stimulation with adenovirus-infected cells and IL-2 is repeated every week to induce CTLs. Whether CTLs induced by the above procedure exhibit specificity to candidate epitope peptides can be assayed using the MHC-tetramer method, ELISPOT assay, chromium release assay, intracellular cytokine staining, or such (Current Protocols in Immunology, Edited by: John E. Coligan, Ada M. Kruisbeek, David H. Margulies, Ethan M. Shevach, Warren Strober, 6.19 ELISPOT Assay to Detect Cytokine-Secreting Murine and Human Cells, 6.24 Detection of Intracellular Cytokines by Flow Cytometry, published by John Wiley & Sons, Inc.).

### (2) Determination of epitope peptide - Method 2

PBMCs isolated from a person with a history of adenovirus infection are suspended at a cell density of 0.1 to 2 x 10⁶ cells/ml in an adequate culture medium, and an arbitrary candidate epitope peptide is added thereto at a concentration of 0.01 to 100 µg/ml. After two days of culture in a 5% CO₂ incubator at 37°C, IL-2 is added. Then, the stimulation with the above peptide and IL-2 is repeated every week or every two weeks to induce CTLs. Whether CTLs induced by the above procedure exhibit specificity to candidate epitope peptides can be assayed using the MHC-tetramer method, ELISPOT assay, chromium release assay, intracellular cytokine staining, or such.

### (3) Determination of epitope peptide - Method 3

PBMCs isolated from a person with an adenovirus infection are suspended at a cell density of 0.1 to 2 x 10⁶ cells/ml in an adequate culture medium, and libraries of synthesized peptides pooled into an appropriate number (for example, ten types of peptides) is added thereto. After two days of culture in a 5% CO₂ incubator at 37°C, IL-2 is added. Then, the stimulation with the pooled peptide and IL-2 is repeated every week or every two weeks to induce CTLs. Whether CTLs induced by the above procedure exhibit specificity to candidate epitope peptides can be assayed using the ELISPOT assay, chromium release assay, intracellular cytokine staining, or such. When a pooled peptide gives a favorable result, a peptide(s) having the ability to induce CTLs can be selected by repeating the above-described experiment using each single peptide. The peptide which reacted is shortened successively to obtain a key peptide as an epitope peptide of the present invention. In general, epitope peptides can be finally shortened to 8 to 10 amino acids.

Specifically, the peptides of the present invention include, for example, peptides comprising the nucleotide sequence of any one of SEQ ID NOs: 1 to 6.

Alternatively, the peptides of the present invention may be peptides comprising any one of the amino acid regions of SEQ ID NOs: 1 to 6. In a preferred embodiment, the peptides of the present invention are peptides in which the adenovirus-specific cytotoxic T cell epitope peptides comprise at least one amino acid sequence selected from the group consisting of the amino acid sequences of SEQ ID NOs: 1 to 6.

Furthermore, the epitope peptides of the present invention may be modified products of the above peptides, so long as their biological and immunological activities are not substantially altered and no adverse activity arises when administered.

In a preferred embodiment, the peptides of the present invention include peptides comprising an amino acid sequence with a substitution, deletion, insertion, and/or addition of one or more amino acids in any one of the amino acid sequences of SEQ ID NOs: 1 to 6, which have a function equivalent to the peptide of any one of SEQ ID NOs: 1 to 6 before modification.

The above-mentioned "equivalent function" includes, for example, (1) the function on the ability to induce adenovirus-specific cytotoxic T cells; or (2) the function on the ability to induce cytotoxic T cells (CTLs) that have T cell receptor capable of specifically recognizing cells presenting a complex with human major histocompatibility antigen (HLA)-A24 molecule, in particular HLA-A*2402, or HLA-Cw*0401 or -Cw*0702 molecule, or the like, on the cell surface. (Herein, peptides having the function described above in (2) are sometimes referred to as antigen peptides restricted by HLA-A24 or the like.)

Whether a modified peptide has a function described above can be evaluated, for example, using the methods described in the Examples herein below, or by the methods with appropriate modifications.

The peptides of the present invention include those that have been modified from their naturally existing states, or remain unmodified. Exemplary modifications include acetylation, acylation, ADP-ribosylation, amidation, covalent attachment of flavin, covalent attachment of heme moiety/moieties, covalent attachment of nucleotides or nucleotide derivatives, covalent attachment of lipids or lipid derivatives, covalent attachment of phosphatidylinositol, cross-linking, cyclization, formation of disulfide bonds, methylation, demethylation, formation of covalent cross-links, formation of cystine, formation of pyroglutamate, formylation, γ-carboxylation, glycosylation, formation of GPI anchors, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Furthermore, the peptides of the present invention may comprise additional amino acid sequences attached to their N or C terminus. In addition, the peptides of the present invention can also be used in the form of a polymer, a complex with saccharides, polyethylene glycol, lipid, and such, or a derivative with radioisotope and such. Alternatively, in the present invention, amino acids include so-called "amino acid analogs". Such amino acid analogs include, for example, N-acylation, O-acylation, esterification, acidamidation, and alkylation products of various amino acids.

Furthermore, a formyl group, acetyl group, t-butoxycarbonyl (t-Boc) group, or such may be linked to the N terminus or the free amino group of a peptide of the present invention and a methyl group, ethyl group, t-butyl group, benzyl group, or such may be linked to the C terminus or the free carboxyl group of a peptide of the present invention, so long as the complex of a molecule, such as HLA-A24, and a peptide of the present invention can be recognized by CTLs that have TCR capable of specifically recognizing cells presenting the complex on the cell surface.

Furthermore, the peptides of the present invention (for example, antigen peptides restricted by HLA-A24 or the like) may be variously modified to facilitate their introduction into the body. A well-known example of such a modification that facilitates introduction into the body is the protein transduction (PT) domain. The PT domain of human immunodeficiency virus (HIV) is a peptide constituted by the amino acids extending from position 49 to 57 (Arg Lys Lys Arg Arg Gln Arg Arg Arg/ SEQ ID NO: 26) of Tat protein. It has been reported that a protein or peptide sequence of interest can be readily introduced into cells by adding the peptide sequence to the protein or peptide sequence before and/or after it (Ryu J, Han K, Park J, Choi SY, Enhanced uptake of a heterologous protein with an HIV Tat protein transduction domains (PTD) at both termini., Mol Cells., 16:385-391 (2003); Kim DT, Mitchell DJ, Brockstedt DG, Fong L, Nolan GP, Fathman CG, Engleman EG, Rothbard JB., Introduction of soluble proteins into the MHC class I pathway by conjugation to an HIV tat peptide., J Immunol., 159:1666-1668 (1997)).

Most of antigens that are presented via an MHC class I molecule are degraded by intracellular proteasomes. Then, the degraded antigens are transferred to TAP (transporter in antigen processing), bind to the complex of MHC class I molecule and β2-microglobulin, which is associated with TAP within rough endoplasmic reticula, and are transported to the cell surface by exocytosis via Golgi apparatuses. Antigen presentation is reported to be effectively achieved when a peptide or protein of interest is fused with HSP (heart shock protein) 70, HSP90, or gp96, which are chaperons acting in the series of antigen-presenting pathway described above (Basu S, Binder RJ, Ramalingam T, Srivastava PK., CD91 is a common receptor for heat shock proteins gp96, hsp90, hsp70, and calreticulin., Immunity., 14:303-313 (2001)). Thus, in one embodiment, the present invention includes proteins arising from the fusion of a peptide of the present invention with the PT domain or a chaperon described above.

Furthermore, the peptides of the present invention can be prepared by various conventional peptide synthesis methods. For example, it is possible to prepare the peptides of the present invention by organic-chemical synthesis methods, such as solid-phase peptide synthesis, or by recombinant DNA techniques by preparing nucleic acids that encode the peptides. The peptides can also be synthesized using a commercially available chemical synthesizer (for example, a peptide synthesizer from Applied Biosystems).

Peptides of the present invention can further be produced by general chemical synthesis methods, according to their amino acid sequences. The methods include peptide synthesis by general liquid phase techniques and solid phase techniques. More specifically, such peptide synthesis methods include stepwise elongation methods, in which respective amino acids are sequentially synthesized one by one according to the amino acid sequence information, to thereby elongate the chain, and fragment condensation methods, in which fragments composed of several amino acids are prepared by prior synthesis, and then the respective fragments are coupled. Either of these methods may be employed to synthesize peptides of the present invention.

Condensation methods employed in the peptide synthesis methods may be performed in accordance with various methods. Specific examples include the azide method, the mixed acid anhydride method, the DCC method, the active ester method, the oxidation-reduction method, the DPPA (diphenylphosphorylazide) method, and the Woodward method.

Solvents generally used in these various methods can be suitably employed. Examples thereof include dimethylformamide (DMF), dimethylsulfoxide (DMSO), hexaphosphoramide, dioxane, tetrahydrofuran (THF), ethyl acetate, and mixtures thereof. In the peptide synthesis reactions described above, a carboxyl group contained in an amino acid or in a peptide that is not involved in the reaction may typically be protected through esterification to form, for example, lower-alkyl esters such as a methyl ester, an ethyl ester, or a tert-butyl ester; a benzyl ester; a p-methoxybenzyl ester; a p-nitrobenzyl ester; or an aralkyl ester. Moreover, amino acids that have a functional group in their side chains, for example, the hydroxyl group of Tyr, may be protected with an acetyl group, a benzyl group, a benzyloxycarbonyl group, or a tert-butyl group. Such protection is not necessarily essential, however. For example, the guanidino group ofArg may be protected with an appropriate protecting group such as a nitro group, a tosyl group, a 2-methoxybenzenesulfonyl group, a methicillen-2-sulfonyl group, a benzyloxycarbonyl group, an isobornyloxycarbonyl group, or an adamantyloxycarbonyl group.

The peptides of the present invention that can be obtained as described above may be suitably purified through conventional methods, such as methods that are customarily used in the field of peptide chemistry, including ion exchange resin methods, partition chromatography, gel chromatography, affinity chromatography, high performance liquid chromatography (HPLC), and countercurrent distribution approaches.

Peptides of the present invention can also be obtained through genetic engineering techniques in which a DNA nucleic acid molecule encoding the peptides of the present invention is synthesized, and then introduced into an appropriate expression vector, and expressed in a host cell.

As an example, first, nucleic acids encoding any one of the amino acid sequence of SEQ ID NO: 1 to 6 is synthesized. Examples of suitable methods include chemical synthesis methods such as a phosphotriester method, a phosphoamidite method (J. Am. Chem. Soc., 89, 4801,1967; and 91, 3350, 1969; Science, 150, 178, 1968; Tetrahedron Lett., 22, 1859, 1981; and 24, 245, 1983), and combinations of such methods. More specifically, the DNA can also be chemically synthesized through a phosphoramidite method or a triester method, and may use a commercially available automated device for polynucleotide synthesis. A double-stranded fragment can also be obtained from chemically synthesized single-stranded products, by synthesizing complementary strands and then by annealing the strands under appropriate conditions, or by using an appropriate primer sequence together with a DNA polymerase to add the complementary strand.

As described above, peptides of the present invention include peptides functionally equivalent to the epitope peptides (SEQ ID NOs: 1 to 6) identified herein.

To prepare a peptide functionally equivalent to another peptide, for example, methods for introducing mutations into amino acids of peptides are well known to those skilled in the art. Specifically, those skilled in the art can prepare a peptide functionally equivalent to peptides comprising any one of the amino acid sequence of SEQ ID NOs: 1 to 6 by introducing appropriate mutations into the original sequence using site-directed mutagenesis (Hashimoto-Gotoh T. et al. Gene. 152: 271-275, 1995; Zoller M.J. and Smith M. Methods Enzymol. 100: 468-500, 1983; Kramer W. et al. Nucleic Acids Res. 12: 9441-9456, 1984; Kramer W. and Fritz H.J. Methods Enzymol. 154: 350-367, 1987; Kunkel T.A. Proc. Natl. Acad. Sci. USA 82: 488-492, 1985; Kunkel T.A. Methods Enzymol. 85: 2763-2766, 1988). Amino acid mutations in peptides may also occur in nature. Thus, both artificially synthesized and naturally occurring proteins comprising an amino acid sequence in which one or more amino acid sequence in the sequence of the epitope peptides (SEQ ID NOs: 1 to 6) identified herein are mutated, and which is functionally equivalent to the epitope peptides, are also included in the present invention.

Examples of the objectives of the above-described amino acid modification (alteration) include:
1. Modification to increase the affinity with HLA (Rosenberg SA, Yang JC, Schwartzentruber DJ, Hwu P, Marincola FM, Topalian SL, Restifo NP, Dudley ME, Schwarz SL, Spiess PJ, Wunderlich JR, Parkhurst MR, Kawakami Y, Seipp CA, Einhorn JH, White DE. Immunologic and therapeutic evaluation of a synthetic peptide vaccine for the treatment of patients with metastatic melanoma, Nat Med. 1998;4:321-327, Berzofsky JA, Ahlers JD, Belyakov IM, Strategies for designing and optimizing new generation vaccines, Nat Rev Immunol. 2001;1:209-219);
2. Modification to improve the recognition of TCR (Fong L, Hou Y, Rivas A, Benike C, Yuen A, Fisher GA, Davis MM, Engleman EG., Altered peptide ligand vaccination with Flt3 ligand expanded dendritic cells for tumor immunotherapy., Proc Natl Acad Sci U S A., 2001;98:8809-8814, Rivoltini L, Squarcina P, Loftus DJ, Castelli C, Tarsini P, Mazzocchi A, Rini F, Viggiano V, Belli F, Parmiani G., A superagonist variant of peptide MART1/Melan A27-35 elicits anti-melanoma CD8+ T cells with enhanced functional characteristics: implication for more effective immunotherapy., Cancer Res., 1999;59:301-306); and
3. Modification to avoid metabolism by peptidases and such in serum (Berzofsky JA, Ahlers JD, Belyakov IM., Strategies for designing and optimizing new generation vaccines., Nat Rev Immunol., 2001;1:209-219, Parmiani G, Castelli C, Dalerba P, Mortarini R, Rivoltini L, Marincola FM, Anichini A., Cancer immunotherapy with peptide-based vaccines: what have we achieved? Where are we going?, J Natl Cancer Inst., 2002;94:805-818, Brinckerhoff LH, Kalashnikov VV, Thompson LW, Yamshchikov GV, Pierce RA, Galavotti HS, Engelhard VH, Slingluff CL Jr., Terminal modifications inhibit proteolytic degradation of an immunogenic MART-1(27-35) peptide: implications for peptide vaccines., Int J Cancer. 1999;83:326-334).

In the mutants described above, the number of mutated (substituted, inserted, deleted, and such) amino acids is not limited, so long as the function of the peptides of the present invention is retained, and the number is typically 5 or less, preferably 4 or less, more preferably 3 or less, and even more preferably 1-2 amino acids. Furthermore, when the above alteration involves, for example, an addition of amino acid residues to the terminus of the peptides of any one of SEQ ID NO: 1 to 6, the number of added amino acids is not limited so long as the function of the peptides of the present invention is retained, and the number is usually 20 or less, preferably 10 or less, and more preferably 5 or less, even more preferably 3 or less, and most preferably, 1-2 amino acids.

In mutating an amino acid, it is preferable to change it into another amino acid (amino acid similar to the unmodified amino acid) that allows the properties of the unmodified amino acid to be conserved. Examples of amino acid side chain characteristics include: side chains having hydrophobic amino acids residues (A, I, L, M, F, P, W, Y, V), hydrophilic residues (R, D, N, C, E, Q, G, H, K, S, T), residues with an aliphatic side chain (G, A, V, L, I, P), residues with a side chain containing a hydroxyl group (S, T, Y), residues with a side chain containing sulfur (C, M), residues with a side chain containing a carboxylic acid and amide group (D, N, E, Q), basic residues (R, K, H), and aromatic residues (H, F, Y, W) (amino acids are shown using the standard one-letter code in the parentheses).

Peptides having a modified amino acid sequence, in which one or more amino acids are deleted, added, and/or substituted with another amino acid, are known to maintain the original biological function (activity) (Mark D.F. et al. Proc. Natl. Acad. Sci. USA 81: 5662-5666, 1984; Zoller M.J. and Smith M. Nucleic Acids Res. 10: 6487-6500, 1982; Wang A. et al. Science 224: 1431-1433; Dalbadie-McFarland G et al: Proc. Natl. Acad. Sci. USA 79: 6409-6413, 1982).

Peptides in which multiple amino acid residues are added to the amino acid sequence of a peptide of the present invention include fusion peptides comprising such a peptide. Such fusion peptides arise from fusion between the peptides of the present invention and other peptides. To produce a fusion protein, a polynucleotide encoding a peptide of the present invention (for example, the peptides of SEQ ID NOs: 1 to 6) and a polynucleotide encoding another peptide are ligated so that their frames match, and inserted into an expression vector to express in a host. Methods known to those skilled in the art can be used. The peptide that is to be fused with a peptide of the present invention is not particularly limited, and includes, for example, β2-microglobulin. That is, fusion peptides between β2-microglobulin and a peptide of the present invention are also included in the present invention.

Other peptides that can be used as peptides that are fused to the peptides of the present invention can be suitably selected according to various objectives, including, for example, for isolating and purifying the peptide or for applied research, in addition to the objective of inducing CTL. Known peptides that can be used as peptides that are fused to the peptides of the present invention include, for example, FLAG (Hopp, T. P. et al., Biotechnology 6, 1204-1210, 1988), 6x His containing six histidine (His) residues, lOx His, influenza agglutinin (HA), human c-myc fragment, VSP-GP fragment, p18HIV fragment, T7-tag, HSV-tag, E-tag, SV40T antigen fragment, lck tag; α-tubulin fragment, B-tag, Protein C fragment, and the like. Examples of proteins that may be fused to peptides of the present invention include GST (glutathione-S-transferase), HA (influenza agglutinin), immunoglobulin constant region, β-galactosidase, MBP (maltose-binding protein), and such. Fusion peptides can be prepared by fusing commercially available polynucleotides, encoding the fusion peptides or peptides discussed above, with polynucleotides encoding the peptides of the present invention, and expressing the prepared fused polynucleotides.

When a fusion comprising a peptide of the present invention is prepared, a peptide sequence which is a protease cleavage site, such as Factor Xa, enterokinase, or thrombin, may be inserted at the junction of fusion. In this case, the peptide is expressed as a peptide fusion in which a purification tag, such as GST, MBP, or FLAG tag described above, is added. After purification, regions other than the desired peptide of the present invention can be cleaved and removed from the peptide fusion by a corresponding protease, such as Factor Xa, enterokinase, or thrombin, if required. Such a treatment is useful.

Other methods for preparing peptides functionally equivalent to a certain peptide which are well known to those skilled in the art include methods using hybridization techniques (Sambrook, J et al., Molecular Cloning 2nd ed., 9.47-9.58, Cold Spring Harbor Lab. press, 1989). Generally, those skilled in the art can isolate polynucleotides highly homologous to polynucleotides encoding the peptides of the present invention based on the polynucleotides or portions thereof from samples of polynucleotides derived from various organisms (for example, derived from adenovirus), artificially synthesized peptide libraries, or others, and isolate peptides which are functionally equivalent to the peptides of the present invention using the polynucleotides.

The present invention includes peptides encoded by a polynucleotide that hybridizes to a polynucleotide encoding a peptide of the present invention shown in SEQ ID NOs: 1 to 6, and which is functionally equivalent to the peptide of any one of SEQ ID NOs: 1 to 6.

The conditions for hybridization used for isolating a polynucleotide encoding a peptide functionally equivalent to the peptide of SEQ ID NOs: 1 to 6 can be appropriately selected by those skilled in the art. For example, low stringency conditions may be used for hybridization. Low stringency conditions are post-hybridization washing in 0.1x SSC, 0.1% SDS at 42°C, for example, and preferably in 0.1x SSC, 0.1% SDS at 50°C. Highly stringent conditions are more preferable, which are washing in 5x SSC, 0.1% SDS at 65°C, for example. Under these conditions, a DNA having a higher homology can be efficiently obtained by increasing the temperature. Multiple factors including the temperature, salt concentration, and such are considered to affect the stringency of hybridization; one skilled in the art can achieve similar stringencies by appropriately selecting these factors.

Furthermore, by using a gene amplification technique (PCR)(Current Protocols in Molecular Biology; editor Ausubel et al.; John Wiley & Sons, Sections 6.1-6.4, 1987) in place of hybridization, polynucleotide fragments that are highly homologous to a polynucleotide encoding the peptides of the present invention (SEQ ID NOs: 1 to 6) can be isolated using primers the design of which is based on portions of the polynucleotide encoding the peptide identified by the present inventors, to obtain a peptide that is functionally equivalent to the peptide identified by the present inventors based on the polynucleotide.

Normally, a peptide encoded by a polynucleotide isolated using the above hybridization techniques or by gene amplification, and which is functionally equivalent to the peptides of SEQ ID NOs: 1 to 6, will have a high homology with the peptides at the amino acid level. The peptides of this invention include peptides functionally equivalent to the peptides of SEQ ID NOs: 1 to 6, and having high homologies with the peptides at the amino acid level. High homology normally means an identity of at least 50% or more at the amino acid level, preferably 75% or more, more preferably 85% or more, and most preferably 95% or more (for example, 96% or more, 97% or more, 98% or more, and 99% or more). Homology between peptides can be determined according to an algorithm described in the literature (Wilbur W. J. and Lipman D. J. Proc. Natl. Acad. Sci. USA 80: 726-730, 1983).

The identity of amino acid sequences can be determined, for example, using the BLAST algorithm of Karlin and Altschul (Proc. Natl. Acad. Sci. USA 87: 2264-2268, 1990; and Proc. Natl. Acad. Sci. USA 90: 5873-5877, 1993). Based on this algorithm, a program called BLASTX has been developed (Altschul et al. J. Mol. Biol. 215: 403-410, 1990). When amino acid sequences are analyzed using BLASTX, parameters are set, for example as follows: score = 50 and wordlength = 3. When BLAST and Gapped BLAST programs are used, default parameters of each program may be used. The specific procedures of these analytic methods are known (http://www.ncbi.nlm.nih.gov.).

The peptides of the present invention can be prepared as recombinant peptides or natural peptides using methods well known to those skilled in the art. A recombinant peptide can be prepared, for example, by inserting a polynucleotide that encodes a peptide of the present invention into an appropriate expression vector; introducing the vector into an appropriate host cell; collecting the recombinant thus obtained; obtaining an extract thereof; and purifying the peptide by subjecting the extract to a chromatographic procedure. Examples of chromatographic procedures are ion exchange chromatography, reverse phase chromatography, gel filtration, or affinity chromatography utilizing a column to which antibodies against peptides of the present invention are immobilized, or combinations of more than one of the aforementioned columns.

When the peptides are expressed within host cells (for example, animal cells or *Escherichia coli (E. coli*)) as recombinant peptides fused with a tag for purification (for example, histidine tag), the expressed fusion peptides can be purified using a commercially available purification column corresponding to the fused tag (nickel column when histidine tag is used).

A natural peptide can be isolated by methods well known to those skilled in the art, for example, through purification by applying extracts of tissues or cells expressing the peptide of the present invention onto an affinity column in which an antibody that binds to the peptide has been attached. The antibody may be a polyclonal or monoclonal antibody.

The above-described nucleic acids and vectors encoding a peptide of the present invention are also included in the present invention.

The nucleic acids (polynucleotides) of the present invention may be in any form, so long as they can encode a peptide of the present invention. Specifically, cDNAs synthesized from mRNAs, genomic DNAs, and chemically-synthesized DNAs can be used. In addition, polynucleotides having any nucleotide sequence based on the degeneracy of genetic code are included as long as they can encode a peptide of the present invention.

The nucleic acids (polynucleotides) of the present invention can be obtained by methods known to those skilled in the art. The nucleic acids can be appropriately produced, for example, using commercially available nucleic acid synthesizers. The nucleotide sequences of prepared polynucleotides can be determined by known methods, for example, the dideoxy nucleotide chain termination method.

Nucleic acids encoding a peptide of the present invention are important, for example, to produce the peptide of the present invention in hosts using genetic recombination techniques. In this case, it is preferred that amino acid codons are modified so that they agree with the codon usage in the host in which the peptide is to be produced, because the frequency of amino acid codon usage varies between hosts. Nucleic acids encoding a peptide of the present invention can be used as vaccines, and can be delivered as naked nucleic acids or using appropriate viral or bacterial vectors (Berzofsky JA, Ahlers JD, Janik J, Morris J, Oh S, Terabe M, Belyakov IM, Progress on new vaccine strategies against chronic viral infections., J Clin Invest., 114:450-462 (2004); Berzofsky JA, Terabe M, Oh S, Belyakov IM, Ahlers JD, Janik JE, Morris JC., Progress on new vaccine strategies for the immunotherapy and prevention of cancer., J Clin Invest., 113:1515-1525 (2004)). Such appropriate bacterial vectors include bacterial vectors of *Salmonella* subspecies. Appropriate viral vectors include, for example, retroviral vectors, adenoviral vectors, Sendai virus vectors, lentiviral vectors, and vaccinia vectors. An example of an appropriate vaccinia vector is modified vaccinia Ankara vectors.

In a preferred embodiment, the nucleic acids of the present invention include, for example, vectors capable of expressing the peptides of the present invention. In general, the vectors carry a DNA construct having a structure in which a nucleic acid of the present invention is operatively linked downstream of a promoter. The vectors commonly used include plasmids, viral vectors, and the like.

Those skilled in the art are capable of appropriately producing such a vector, carrying the desired DNA, using common genetic engineering techniques. Normally, various commercially available expression vectors can be used.

The vectors of the present invention are also useful for retaining a polynucleotide of the present invention in a host cell or for expressing the peptides of the present invention in a host cell. A nucleic acid (polynucleotide) of the present invention is normally retained (inserted) in an appropriate vector before being introduced into a host cell. The vectors are not particularly limited, so long as the vectors can stably retain the inserted DNA. For instance, when *E*. *coli* is used as the host, vectors such as pBluescript (Stratagene), and the like are preferred cloning vectors, although a variety of commercially available vectors may be used. If a vector is used for the purpose of producing peptides of the present invention, expression vectors are especially useful. The expression vectors are not particularly limited so long as they express the peptide *in vitro,* in *E. coli,* in culture cells, or *in vivo.* Examples thereof include pBEST vector (Promega) for *in vitro* expression, pET vector (Invitrogen) for expression in *E. coli,* pME18S-FL3 vector (GenBank Accession No. AB009864) for expression in cultured cells, and pME18S vector (Mol Cell Biol. 8: 466-472, 1988) for *in vivo* expression. Insertion of nucleic acids of the present invention into a vector can be performed by customary methods, such as ligase reactions using restriction enzyme sites.

There is no particular limitation on the host cell, and various host cells may be used according to the desired purpose. Examples of cells that express peptides include bacterial cells (such as those of *Streptococcus, Staphylococcus, E. coli, Streptomyces,* and *Bacillus subtilis*), insect cells (such as *Drosophila* S2 and *Spodoptera* SF9), animal cells (such as CHO, COS, HeLa, C127, 3T3, BHK, HEK293, and Bowes melanoma cells), and plant cells. Vectors can be introduced into a host cell by well known methods, including, for example, the calcium phosphate precipitation method, the electroporation method (Current Protocols in Molecular Biology; editor: Ausubel et al., 1987; Publisher: John Wiley & Sons. Section 9.1-9.9), the lipofection method (GIBCO-BRL), and the microinjection method.

Appropriate secretion signals may be incorporated into the peptide of interest such that the peptide that has been expressed in the host cell is secreted into the lumen of the endoplasmic reticulum, into the periplasmic space, or into the extracellular environment. These signals may be endogenous to the peptide of interest or may be heterologous signals.

Regarding the collection of the peptides in the context of the above production methods, the medium is collected if the peptide of the present invention is secreted into the medium. If the peptide of the present invention is produced within cells, the cells are first lysed before the peptide is collected.

The peptides of the present invention can be collected and purified from recombinant cell cultures by well-known methods, including ammonium sulfate or ethanol precipitation, acid extraction, anion or cation exchange chromatography, phosphocellulose chromatography, hydrophobic interaction chromatography, affinity chromatography, hydroxylapatite chromatography, and lectin chromatography.

Moreover, methods for expressing the polynucleotides (nucleic acid) of the present invention in the living body of an animal include methods in which the nucleic acid of the present invention is incorporated into an appropriate vector and the vector is introduced into the living body by the retrovirus method, the liposome method, the cationic liposome method, the adenovirus method, or the like. In this way, immunotherapy can be performed. Examples of the vectors to be used include retroviral vectors, but are not limited thereto. General gene manipulations such as the insertion of the DNA of the present invention into a vector can be performed in accordance with customary methods (Molecular Cloning, 5.61-5.63). The administration into the living body can be performed either by the *ex vivo* method or the *in vivo* method.

The peptides of the present invention (adenovirus-specific CTL epitope peptides) can be used as peptide vaccines in active immunotherapy. More specifically, vaccines comprising CTL epitope peptides of the present invention can be administered to a healthy individual or to a patient so that the adenovirus-specific CTLs proliferate in the body, to thereby be useful in preventing or treating the diseases. Depending on the purpose of vaccination, an epitope peptide can be used alone or two or more different types of peptides can be mixed and used in combination.

Thus, the present invention provides vaccines for treating or preventing adenovirus infection, which include, as an active ingredient, peptides or nucleic acids of the present invention.

The "vaccines" of the present invention can also be referred to as "active immunotherapeutic agents", "immunotherapeutic agents", or "therapeutic agents for adenovirus-associated diseases". Alternatively, herein, the "therapeutic agents" can also be referred to as "pharmaceuticals", "pharmaceutical compositions", "therapeutic medicines", or the like.

Furthermore, antigen-presenting cells that present CTL epitope peptides of the present invention can be used as vaccines in active immunotherapy. The antigen-presenting cells that present CTL epitope peptides refers to:
1. epitope peptide-pulsed antigen-presenting cells, which are prepared by mixing antigen-presenting cells with the CTL epitope peptide in an adequate culture medium for 30 minutes to one hour;
2. antigen-presenting cells allowed to present a CTL epitope peptide by gene transfer and such using a nucleic acid encoding the CTL epitope peptide;
3. artificially produced antigen-presenting cells having the ability to present an antigen; and such.

The antigen-presenting cells refer, for example, to dendritic cells, B cells, macrophages, and certain types of T cells, which express HLA capable of binding with the peptides on their surface and have the ability to stimulate CTLs. The artificially produced antigen-presenting cells having the ability to present an antigen can be prepared, for example, by immobilizing the ternary complex of HLA, CTL epitope peptide, and β2-microglobulin onto lipid bilayer, plastic or latex beads, or such, and immobilizing a costimulatory molecule, such as CD80, CD83, or CD86, which can stimulate CTLs, or an antibody or the like, which act agonistically to CD28, a T cell ligand that binds to the costimulatory molecules (Oelke M, Maus MV, Didiano D, June CH, Mackensen A, Schneck JP., Ex vivo induction and expansion of antigen-specific cytotoxic T cells by HLA-Ig-coated artificial antigen-presenting cells., Nat Med. 9:619-624 (2003); Walter S, Herrgen L, Schoor O, Jung G, Wernet D, Buhring HJ, Rammensee HG, Stevanovic S., Cutting edge: predetermined avidity of human CD8 T cells expanded on calibrated MHC/anti-CD28-coated microspheres., J Immunol. 171:4974-4978 (2003); Oosten LE, Blokland E, van Halteren AG, Curtsinger J, Mescher MF, Falkenburg JH, Mutis T, Goulmy E., Artificial antigen-presenting constructs efficiently stimulate minor histocompatibility antigen-specific cytotoxic T lymphocytes., Blood. 104:224-226 (2004)).

Thus, in a preferred embodiment, the present invention provides vaccines including, as an active ingredient, antigen-presenting cells that present a peptide of the present invention by HLA.

The nucleic acids of the present invention can be used as DNA vaccines, recombinant viral vector vaccines, or such in active immunotherapy. In this case, it is preferred that the nucleotide sequences of CTL epitope peptides are altered to be compatible with the codon usage in the host in which the recombinant vaccines or recombinant viral vaccines are produced (Casimiro, D.R. et al., Comparative Immunogenicity in Rhesus Monkeys of DNA Plasmid, Recombinant Vaccinia Virus, and Replication-Defective Adenovirus Vectors Expressing a Human Immunodeficiency Virus Type 1 gag Gene, J. Virol., 77:6305-6313 (2003); Berzofsky JA, Ahlers JD, Janik J, Morris J, Oh S, Terabe M, Belyakov IM., Progress on new vaccine strategies against chronic viral infections., J Clin Invest., 114:450-462 (2004)).

Vaccines including a peptide of the present invention or antigen-presenting cells that present a peptide of the present invention can be prepared by methods known in the technical field. Such vaccines include, for example, agents, such as injections and solid agents, including, as an active ingredient, a peptide of the present invention.

The peptides of the present invention can be used in producing passive immunotherapeutic agents against adenovirus. Adenovirus-specific CTLs obtained by the procedure described herein below are suspended in PBS containing human albumin or the like, and used as passive immunotherapeutic agents against adenovirus. The adenovirus-specific CTLs included in the passive immunotherapeutic agents can be obtained by the preparation methods described below. CTLs can also be used after purification to improve the purity.

Specific methods for preparing CTLs are described below. However, methods for preparing CTLs are not limited to these methods.

### (a) Preparation of CTLs - Method 1

PBMCs are reacted with an adequate concentration of an adenovirus-specific MHC-tetramer reagent. Adenovirus-specific CTLs bound to the MHC-tetramer reagent are stained with a labelling dye. Thus, stained CTLs alone are isolated using a cell sorter, microscope, or such. The resulting isolated adenovirus-specific CTLs are stimulated for growth with a T cell stimulator, such as anti-CD3 antibody, PHA, or IL-2, or with antigen-presenting cells whose growth potential has been eliminated by X-ray irradiation, mitomycin treatment, or such, to obtain the required number of cells for passive immunotherapy.

### (b) Preparation of CTLs - Method 2

An adenovirus-specific MHC monomer and/or MHC tetramer reagent is solid-phased on a sterile plate or such, and PBMCs are cultured in the solid-phased plate. To isolate adenovirus-specific CTLs that are bound to the MHC monomer and/or MHC tetramer, which is solid-phased on the plate, unbound floating cells are washed off, and then only the antigen-specific CTLs remaining on the plate are suspended in a fresh culture medium. The resulting isolated adenovirus-specific CTLs are stimulated for growth with a T cell stimulator, such as anti-CD3 antibody, PHA, or IL-2, or with antigen-presenting cells whose growth potential has been eliminated by X-ray irradiation, mitomycin treatment, or such, to obtain the required number of cells for passive immunotherapy.

### (c) Preparation of CTLs - Method 3

An adenovirus-specific MHC monomer and/or MHC tetramer reagent and a costimulatory molecule, such as CD80, CD83, or CD86, or an antibody or the like, which act agonistically to CD28, a T cell ligand that binds to the costimulatory molecule are solid-phased on a sterile plate or such, and PBMCs are cultured in the solid-phased plate. After two days, IL-2 is added to the culture medium. The cells are cultured in a 5% CO₂ incubator at 37°C for seven to ten days. The cultured cells were collected, and then further cultured in a fresh solid-phased plate. This process is repeated to obtain the required number of CTLs for passive immunotherapy.

### (d) Preparation of CTLs - Method 4

PBMCs or T cells are stimulated directly with a CTL epitope peptide of the present invention, or with antigen-presenting cells pulsed with the peptide, antigen-presenting cells introduced with the gene, or artificially produced antigen-presenting cells having the ability to present the antigen. CTLs induced by the stimulation are cultured in a 5% CO₂ incubator at 37°C for seven to ten days. The stimulation with the CTL epitope peptide and IL-2, or with the antigen-presenting cells and IL-2 is repeated every week to obtain the required number of CTLs for passive immunotherapy.

MHC-monomers and MHC-tetramers using the peptides of the present invention (adenovirus-specific CTL epitope peptides) can be prepared by known methods (US Patent Number 5,635,363, Inventors: J. D. Altman, M. G. McHeyzer-Williams, Mark M. Davis, Compositions and methods for the detection, quantitation and purification of antigen-specific T cells; French Application Number FR9911133, Inventor: M. Bonneville, *et al.,* Means for detecting and for purifying CD8+ T-lymphocyte populations specific for peptides presented in the HLA context). The MHC-monomer, which is the complex of HLA class I molecule purified from a genetically modified host for protein expression, β2-microglobulin, and a CTL epitope peptide of the present invention, is formed in a buffer. A biotin-binding site is added to the C terminus of the recombinant HLA class I molecule beforehand. After MHC-monomer formation, biotin is allowed to bind to this site. The MHC-tetramer can be prepared by combining commercially available dye-labeled streptavidin with biotinylated MHC-monomer at a molar ratio of 1:4.

When the proportion of specific CTLs is low in the methods for preparing CTLs, highly pure CTLs can be collected by the methods described below, if required.

### (a) Purification using MHC-tetramer reagent

An adenovirus-specific MHC-tetramer reagent is reacted with CTLs induced by the methods for preparing CTLs. The specific CTLs can be isolated using a magnetically-labeled secondary antibody against a labelling dye that labels the MHC-tetramer. Such magnetically-labeled secondary antibodies and magnetic cell separation devices are available from Dynal Co. or Miltenyi Biotec GmbH. The resulting isolated adenovirus-specific CTLs are stimulated for growth with a T cell stimulator, such as anti-CD3 antibody, PHA, or IL-2, to obtain the required number of cells for passive immunotherapy.

### (b) Purification using secreted cytokines

Adenovirus-specific CTLs can be purified by using cytokines or the like released from adenovirus-specific CTLs. For example, using a kit available from Miltenyi Biotec GmbH, cytokines released from CTLs are captured by specific antibodies on the cell surface. The cells are stained with cytokine-specific labeled antibodies and then with a magnetically labeled label-specific antibody. Then, the cells can be purified using a device for separating magnetically labeled cells. The resulting isolated adenovirus-specific CTLs are stimulated for growth with a T cell stimulator, such as anti-CD3 antibody, PHA, or IL-2, to obtain the required number of cells for passive immunotherapy.

### (c) Purification using cell surface protein-specific antibodies

It is reported that on the cell surface of specific CTLs, the expression of some cell surface proteins (for example, CD107a, CD107b, CD63, CD69, etc.) is enhanced upon stimulation with specific peptides (Betts MR, Brenchley JM, Price DA, De Rosa SC, Douek DC, Roederer M, Koup RA., Sensitive and viable identification of antigen-specific CD8+ T cells by a flow cytometric assay for degranulation., J Immunol Methods., 281:65-78 (2003); Trimble LA, Shankar P, Patterson M, Daily JP, Lieberman J., Human immunodeficiency virus-specific circulating CD8 T lymphocytes have down-modulated CD3zeta and CD28, key signaling molecules for T-cell activation., J Virol., 74:7320-7330 (2000)). By magnetically labelling specific antibodies against such proteins, CTLs can be purified using a magnetic separation device or such. Alternatively, CTLs can also be purified, by magnetically labelling anti-IgG antibodies or such against the specific antibody. Alternatively, specific CTLs can also be purified by coating a plastic culture plate with such an antibody, culturing stimulated PBMCs in the plate, and washing off the groups of cells that are unbound to the plate. The resulting isolated adenovirus-specific CTLs are stimulated for growth with a T cell stimulator, such as anti-CD3 antibody, PHA, or IL-2, to obtain the required number of cells for passive immunotherapy.

The present invention provides passive immunotherapeutic agents against adenovirus (immunotherapeutic agents) including, as an active ingredient, cytotoxic T cells (CTLs) obtained and purified as described above.

The "passive immunotherapeutic agents" of the present invention can also be referred to as "immunotherapeutic agents", "therapeutic agents for adenovirus-associated diseases", or "CTL inducers".

In a preferred embodiment of the present invention, the passive immunotherapeutic agents are passive immunotherapeutic agents against adenovirus including, as an active ingredient, adenovirus-specific cytotoxic T cells that are obtained by stimulating peripheral blood lymphocytes with the peptides of the present invention or antigen-presenting cells that present the peptides by HLA.

In another embodiment, the passive immunotherapeutic agents against adenovirus including, as an active ingredient, cytotoxic T cells that are obtained by reacting peripheral blood lymphocytes with the major histocompatibility antigen complex and/or major histocompatibility antigen complex-tetramer prepared from the peptides of the present invention, allowing the formation of a complex in which the major histocompatibility antigen complex and/or major histocompatibility antigen complex-tetramer are bound with cytotoxic T cells, and isolating the cytotoxic T cells from the complex.

The agents of the present invention (the vaccines, passive immunotherapeutic agent and such of the present invention) can be combined with a physiologically acceptable carrier, excipient, diluent, or the like, and then administered orally or parenterally as pharmaceutical compositions. The dosage form for oral agents may be granules, powders, tablets, capsules, solutions, emulsions, suspensions, or the like. The dosage form for parenteral agents may be selected from injections, drops, external medicines, suppositories, or the like. Examples of injections include agents for subcutaneous injection, intramuscular injection, and intraperitoneal injection. Examples of external medicines include agents for nasal administration and ointments. Preparation methods for preparing the above dosage forms to include agents of the present invention as the main ingredients are known.

For example, tablets for oral administration can be produced by mixing the agents of the present invention with excipients, disintegrants, binders, lubricants, and the like, and by compression and shaping.

For example, antigen-presenting cells that present the peptides of the present invention can be used in combination with pharmaceutically acceptable excipients that do not affect the activity of the peptides or the cells. Examples include, water, saline, dextrose, ethanol, glycerol, dimethyl sulphoxide (DMSO), and other adjuvants or mixtures thereof. Furthermore, auxiliary agents, such as albumin, wetting agent, or emulsifying agent can be added if needed. Commonly used disintegrants include calcium carbonate, carboxymethylcellulose calcium, and the like. Binders include gum arabic, carboxymethylcellulose, and polyvinylpyrrolidone. Known lubricants include talc, magnesium stearate, and such.

Tablets including the agents of the present invention can be masked or coated to form enteric preparations by known methods. Ethylcellulose, polyoxyethyleneglycol, and the like may be used as coating agents.

Moreover, injectable preparations can be obtained by mixing the agents of the present invention, serving as the main ingredients, with an appropriate dispersing agent, or by dissolving or dispersing them in a dispersion medium. The agents may be in the form of either an aqueous preparation or an oil-based preparation by appropriate selection of dispersion medium. Distilled water, physiological saline, Ringer's solution, or the like is used as dispersion media when preparing aqueous preparations. Various vegetable oils, propylene glycol, or the like, is used as dispersion media for oil-based preparations. Preservatives such as paraben may also be added as required. Moreover, publicly known isotonizing agents such as sodium chloride and glucose can be added to the injectable preparations. Furthermore, soothing agents such as benzalkonium chloride and procaine hydrochloride can be added.

Moreover, external preparations can be produced by forming the agents of the present invention into solid, liquid, or semisolid compositions. In the case of solid or liquid compositions, external preparations can be produced by making compositions similar to those described above. Semisolid compositions can be prepared by adding thickeners to appropriate solvents as required. Water, ethyl alcohol, polyethylene glycol, or the like can be used as solvents. Bentonite, polyvinyl alcohol, acrylic acid, methacrylic acid, polyvinylpyrrolidone, or the like are commonly used as thickeners. Preservatives such as benzalkonium chloride can be added to these compositions. Moreover, these compositions can also be combined with oil bases such as cacao butter or with aqueous gel bases such as cellulose derivatives as carriers, to prepare suppositories.

Furthermore, the peptides of the present invention can be prescribed in neutral or salt form. Examples of pharmaceutically acceptable salts include inorganic salts such as hydrochloric acid and phosphoric acid, and organic salts such as acetic acid and tartaric acid.

If the agents of the present invention are used as agents for gene therapy, there are methods of directly administering the agents of the present invention by injection, and methods of administering vectors incorporating a nucleic acid. Examples of the vectors include adenoviral vectors, adeno-associated viral vectors, herpesvirus vectors, vaccinia virus vectors, retroviral vectors, and lentiviral vectors. The use of such viral vectors enables efficient administration of therapeutic agents.

Moreover, it is possible to introduce the agents of the present invention into phospholipid vesicles such as liposomes and then to administer the vesicles. For example, vesicles retaining the peptides or vectors of the present invention are introduced into given cells by the lipofection method. Cells thus obtained are systemically administered into a vein or an artery, or the like.

The agents of the present invention can be administrated parenterally or orally. When the chief ingredient in the agents is a peptide, in general, parenteral administration is preferred. Parenteral administration includes nasal administration, injection such as subcutaneous, intramuscular, or intravenous injection, suppository, etc. Meanwhile, for oral administration, the agents can be prepared as mixtures with excipients such as starch, mannitol, lactose, magnesium stearate, and cellulose.

The vaccines of the present invention are administered at a therapeutically effective dose. The dose depends on the subject to be treated and the immune system. Necessary doses are determined by physicians. Typically, the appropriate dose is 1 mg to 100 mg of the peptide of the present invention (epitope peptide) per patient, or 10⁶ to 10⁹ epitope peptide-pulsed cells per patient. Furthermore, the administration intervals can be set according to the subject and purpose.

Furthermore, adenovirus-associated diseases on which an agent of the present invention is expected to produce a preventive or therapeutic effect include, for example, respiratory infections represented by pneumonia; ophthalmic infections represented by pool fever and epidemic keratoconjunctivitis; gastrointestinal infections including gastroenteritis; urogenital infections including hemorrhagic cystitis and urethritis; and others.

Animals that can be inoculated with the agents (vaccines) of the present invention are not particularly limited, as long as they have an immune system and can be infected with adenovirus. The animals are preferably humans.

In another aspect, the present invention provides methods for quantifying (detecting or assaying) adenovirus-specific cytotoxic T cells using the peptides of the present invention.

It is important to know whether adenovirus-specific CTLs are present in peripheral blood of high-risk patients (persons with reduced immunocompetence for some reason, patients with congenital immunodeficiency, patients who receive an immunosuppressant to prevent rejection after transplantation of bone marrow, hematopoietic stem cells, cord blood, or a solid organ, patients with chronic virus infection, AIDS patients, elderly people, babies and infants, pregnant women, etc.), because such information is useful for infection management, including the appropriate use of anti-viral agents and immunosuppressants. Adenovirus-specific CTLs can be quantified, for example, by the following three methods using the peptides of the present invention (CTL epitope peptides); however, such methods are not limited to these methods.

### (a) Quantitation method 1

Adenovirus-specific CTLs in peripheral blood can be quantified by using MHC-tetramer reagents produced using CTL epitope peptides of the present invention. Quantitation can be carried out, for example, by the following procedure. Peripheral blood or PBMCs is reacted with an adequate concentration of an MHC-tetramer reagent. When bound to the MHC-tetramer reagent, CTLs are stained with a labelling dye. Thus, stained CTLs are counted using a flow cytometer, microscope, or such. The T cell subsets of adenovirus-specific CTLs can also be determined at the same time by reacting an anti-CD3 antibody, anti-CD4 antibody, anti-CD8 antibody, or the like, which is labeled with a dye that is different from that of the MHC-tetramer reagent at the time of reaction with the MHC-tetramer reagent.

### (b) Quantitation method 2

This method quantifies cytokines and/or chemokines, such as interferon gamma (IFNγ), tumor necrosis factor (TNF), or interleukin, produced by CTLs upon stimulation of PBMCs with the CTL epitope peptides of the present invention. This method is illustrated below in more detail by using IFNγ as an example parameter.

### b-1. Method based on cytokine quantitation 1 (quantitation of cells producing intracellular IFNγ)

PBMCs are suspended at a cell density of about 2 x 10⁶ cells/ml in an adequate culture medium, and CTL epitope peptides of the present invention are added thereto. Furthermore, an agent that inhibits intracellular protein transport (for example, brefeldin A, monensin, or such) is added, and the cells are cultured in a 5% CO₂ incubator at 37°C for 5 to 16 hours. After culture, the cells are reacted with an antibody against a T cell marker (anti-CD3 antibody, anti-CD4 antibody, or anti-CD8 antibody) and/or an MHC-tetramer reagent. After fixation, the cells are subjected to membrane permeabilization and allowed to react to a dye-labeled anti-IFNγ antibody. The percentage of IFNγ-positive cells in the whole cells, T cells, or MHC-tetramer-positive cells is determined through analysis using a flow cytometer or such.

### b-2. Method based on cytokine quantitation 2 (ELISPOT assay)

PBMCs are plated in a 96-well MultiScreen-HA plate (Millipore Co.) solid phased with an anti-IFNγ antibody. Epitope peptides are aliquoted into each well, and the plate is incubated in a5% CO₂ incubator at 37°C for 20 hours. On the next day, the plate is washed, and incubated with anti-IFNγ antibody and peroxidase-labelled anti-IgG antibody in this order. Then, a peroxidase substrate is added, and IFNγ spots are visualized by color development and counted under a stereoscopic microscope.

### b-3. Method based on cytokine quantitation 3 (quantitation of IFNγ secreted to the culture supernatant)

PBMCs are suspended at a cell density of about 2 x 10⁶ cells/ml in an adequate culture medium, and CTL epitope peptides of the present invention are added thereto. The cells are cultured in a 5% CO₂ incubator at 37°C for 24 to 48 hours. After culture, the supernatant is collected and the concentration of IFNγ therein is determined using a commercially available ELISA kit (for example, HUMAN IFN gamma ELISA, MBL Co.).

### (c) Quantitation method 3

Quantitation is carried out using cell surface protein-specific antibodies. It is reported that expression of cell surface proteins containing for example CD107a, CD107b, CD63, and CD69 is upregulated on antigen-specific CTL upon stimulation with specific peptides. CTLs bound to a labelled antibody are stained with a labelling dye by mixing peptide-stimulated PBMCs or the like with the labeled antibody that specifically recognizes such a protein. Thus, stained CTLs are counted using a flow cytometer, microscope, or such. T cell subsets of adenovirus-specific CTLs can also be determined by reacting simultaneously or in succession an anti-CD3 antibody, anti-CD4 antibody, anti-CD8 antibody, or the like that has been labeled with a dye different from that of the labeled antibody at the time of reaction with the labeled antibody.

In a preferred embodiment, the quantitation methods of the present invention include, specifically, the quantitation methods described in the above (a) to (c).

Thus, preferred embodiments of the quantitation methods of the present invention are methods for quantifying adenovirus-specific cytotoxic T cells, which include stimulating peripheral blood with the peptides of the present invention, collecting cytotoxic T cells specific to the virus, and assaying a cytokine and/or chemokine, and/or cell surface molecule produced by the cytotoxic T cells.

Furthermore, the MHC-tetramer (or monomer or multimer) reagent can be used to quantify specific CTLs as well as to quantify separated, purified specific CTLs as described above. In another embodiment, the present invention relates to methods for quantifying adenovirus-specific cytotoxic T cells in peripheral blood, which include preparing MHC-tetramer reagents from the peptides of the present invention and reacting the MHC-tetramer reagents with peripheral blood.

In the methods described above, peripheral blood samples that have been previously collected or isolated from subjects may be used.

The present invention also provides methods for inducing (activating) cytotoxic T cells, which include inducing cytotoxic T cells using the peptides of the present invention.

Preferred embodiments of the induction methods of the present invention relate to methods for inducing adenovirus-specific cytotoxic T cells (CTL) by contacting peripheral blood mononuclear cells with the peptides of the present invention (for example, antigen peptides restricted by HLA-A24 or the like) in a culture medium containing plasma.

In addition, methods for detecting or quantifying adenovirus-specific CTLs that are induced by the methods described above are also included in the present invention.

Furthermore, adenovirus-specific cytotoxic T cells included by the above-described methods can be used as an ingredient of passive immunotherapeutic agents against adenovirus. Thus, passive immunotherapeutic agents can be produced by inducing adenovirus-specific cytotoxic T cells using the above-described methods, and obtaining the T cells.

The present invention provides methods for producing passive immunotherapeutic agents, which use the method of the present invention for inducing adenovirus-specific cytotoxic T cells.

Preferred embodiments of the production methods are methods that include the step of obtaining adenovirus-specific cytotoxic T cells by stimulating peripheral blood lymphocytes with the peptides of the present invention or with antigen-presenting cells that present the peptides by HLA.

In another preferred embodiment, the methods are for producing passive immunotherapeutic agents against adenovirus, which include the step of obtaining cytotoxic T cells by reacting peripheral blood lymphocytes with the major histocompatibility antigen complex and/or major histocompatibility antigen complex-tetramer prepared from the peptides of the present invention, allowing the formation of a complex in which the major histocompatibility antigen complex and/or major histocompatibility antigen complex-tetramer are bound with cytotoxic T cells, and isolating cytotoxic T cells from the complex.

The production methods described above may include, in addition to the steps described above, the step of "mixing pharmaceutically acceptable carriers to the isolated cytotoxic T cells", if required.

Furthermore, MHC-tetramer reagents prepared using the peptides of the present invention can be used not only to quantify specific CTLs but also to simultaneously assess the differentiation stages and functionality of specific CTLs, by combining with antibodies specific to cell surface proteins or with methods for quantifying cells producing intracellular cytokines.

The present invention also relates to methods for treating or preventing adenovirus infection, which include the step of administering to individuals (for example, patients) any one of: peptides described herein; nucleic acids encoding the peptides; antigen-presenting cells that present the peptides by HLA; adenovirus-specific cytotoxic T cells that are obtained by stimulating peripheral blood lymphocytes with the peptides or the antigen-presenting cells that present the peptides by HLA; and cytotoxic T cells that are obtained by reacting peripheral blood lymphocytes with the major histocompatibility antigen complex and/or major histocompatibility antigen complex-tetramer prepared from the peptides, allowing the formation of a complex in which the major histocompatibility antigen complex and/or major histocompatibility antigen complex-tetramer are bound with cytotoxic T cells, and isolating cytotoxic T cells from the complex. Individuals in the preventive or therapeutic methods of the present invention are not particularly limited, as long as they have an immune system and can be infected with adenovirus. The individuals are preferably humans. The administration to individuals can be performed in accordance with the administration methods as described above for the agents of the present invention.

The present invention also relates to the use of any one of: peptides described herein; nucleic acids encoding the peptides; antigen-presenting cells that present the peptides by HLA, adenovirus-specific cytotoxic T cells that are obtained by stimulating peripheral blood lymphocytes with the peptides or the antigen-presenting cells that present the peptides by HLA; and cytotoxic T cells that are obtained by reacting peripheral blood lymphocytes with the major histocompatibility antigen complex and/or major histocompatibility antigen complex-tetramer prepared from the peptides, allowing the formation of a complex in which the major histocompatibility antigen complex and/or major histocompatibility antigen complex-tetramer are bound with cytotoxic T cells, and isolating the cytotoxic T cells from the complex, in producing vaccines for treating or preventing adenovirus infection or passive immunotherapeutic agents against adenovirus (immunotherapeutic agents).

All prior-art documents cited herein are incorporated herein by reference.

### Examples

Hereinafter, the present invention is described in more detail with reference to Examples; however, it should not be construed as being limited thereto. Unless otherwise stated, experiments were carried out with reference to the following publication: Men-eki Jikken Sousa Hou (Protocols for immunological experiments), Shunsuke Migita, Susumu Konda, Tasuku Honjo, and Toshiyuki Hamaoka, Nankodo Co.

### [Example 1] Selection of candidates for adenovirus-specific CTL epitope peptides

Fifty one serotypes of adenovirus have been reported and are categorized into six subgroups (A to F). Hexon proteins, which exhibit the highest homology among the 51 serotypes, were analyzed to arrive at epitope peptides that specifically recognize 11 types of subgroup B, the group of virus which is particularly problematic in post-transplantation infection, and epitope peptides specific to all adenoviruses including subgroup B. The adenovirus particle has an envelope-free regular icosahedron structure having a diameter of 80 to 90 nm. Hexon forms the 20 triangular facets and ridges in the regular icosahedron.

The selection of candidate epitope peptides specific to adenoviral hexon was conducted for the HLA-A*24 molecule, which is carried by about 60% of Japanese population. Selection was specifically achieved through searches with various software publicly available on the Internet which can be used to retrieve candidate epitope peptides composed of 8 to 10 amino acids and having a binding motif to the HLA-A*24 molecule.

As a result, ten types of candidate CTL epitope peptides composed of 9 amino acids and having an HLA-A*24 molecule-binding motif were selected from the amino acid sequences of hexon of type 11 adenovirus. Peptides of the present invention were subsequently synthesized. The synthesized candidate CTL epitope peptides are listed below. An HLA-A*2402-restricted epitope peptide (TYPVLEEMF (SEQ ID NO: 11)) of EBV BRLF1 protein and HLA-A*2402-restricted epitope peptide (QYDPVAALF (SEQ ID NO: 12)) of HCMV pp65 protein were synthesized as positive control peptides. An HLA-A*2402-restricted peptide (RYLRDQQLL (SEQ ID NO: 13)) of HIV envelope protein was synthesized as a negative control (Kuzushima K, Hayashi N, Kudoh A, Akatsuka Y, Tsujimura K, Morishima Y, Tsurumi T. Tetramer-assisted identification and characterization of epitopes recognized by HLA A*2402-restricted Epstein-Barr virus-specific CD8+ T cells. Blood. 101:1460-1468 (2003)).
Synthesized HLA-A*24-binding peptides of the hexon of type 11 adenovirus
Lys Tyr Thr Pro Ser Asn Val Thr Leu (KYT) (SEQ ID NO: 7)
Asp Tyr Leu Ser Ala Ala Asn Met Leu (DYL) (SEQ ID NO: 1)
Leu Tyr Ala Asn Ser Ala His Ala Leu (LYA) (SEQ ID NO: 2)
Val Tyr Ser Gly Ser Ile Pro Tyr Leu (VYS) (SEQ ID NO: 3)
Thr Tyr Phe Asn Leu Gly Asn Lys Phe (TYF) (SEQ ID NO: 4)
Ser Tyr Gln Leu Leu Leu Asp Ser Leu (SYQ) (SEQ ID NO: 8)
Leu Tyr Ser Asn Val Ala Leu Tyr Leu (LYS) (SEQ ID NO: 5)
Asn Tyr Asn Ile Gly Tyr Gln Gly Phe (NYN) (SEQ ID NO: 9)
Asn Tyr Ile Gly Phe Arg Asp Asn Phe (NYI) (SEQ ID NO: 10)
Gly Tyr Lys Asp Arg Met Tyr Ser Phe (GYK) (SEQ ID NO: 6)

Characteristics of the synthesized HLA-A*24-binding peptides of the hexon of type 11 adenovirus are shown in Table 1. Each peptide name is indicated by an abbreviation using the sequence of the first three amino acids from the N terminus of the synthesized peptide. From the left, the columns show the peptide name, amino acid sequence, the position in the amino acid sequence along the type-11 adenovirus hexon, the number of amino acids, and a score determined in HLA Peptide Binding Predictions (http://thr.cit.nih.gov/molbio/hla_bind/) of BIMAS (BioInformatics & Molecular Analysis Section; http://thr.cit.nih.gov/index.shtml), which was used in the analysis. The score is a value used to predict the affinity between HLA-A*24 and a peptide. The higher the score, the more likely the peptide and HLA are to form a stable complex.

**[Table 1]**

| PEPTIDE NAME | AMINO ACID SEQUENCE^{a} | POSITION | NUMBER OF AMINO ACIDS | SCORE^{b} |
|---|---|---|---|---|
| KYT | KYTPSNVTL (SEQ ID NO: 7) | 482-490 | 9 | 480 |
| SYQ | SYQLLLDSL (SEQ ID NO: 8) | 366-374 | 9 | 360 |
| DYL | DYLSAANML (SEQ ID NO:1) | 641-649 | 9 | 360 |
| LYS | LYSNVALYL (SEQ ID NO:5) | 469-477 | 9 | 280 |
| VYS | VYSGSIPYL (SEQ ID NO:3) | 696-704 | 9 | 200 |
| LYA | LYANSAHAL (SEQ ID NO: 5) | 889-897 | 9 | 200 |
| NYN | NYNIGYQGF (SEQ ID NO: 9) | 769-777 | 9 | 180 |
| TYF | TYFNLGNKF (SEQ ID NO: 4) | 37-45 | 9 | 158 |
| NYI | NYIGFRDNF (SEQ ID NO: 10) | 322-330 | 9 | 150 |
| GYK | GYKDRMYSF (SEQ ID NO: 6) | 782-790 | 9 | 120 |

| | | | | |
|---|---|---|---|---|
| Notes for the symbols a and b in Table 1 above are shown below: a: amino acids of the anchor motifs are indicated with boldface letters. b: the calculated half-times for the dissociation from HLA-A*24 are determined by using a computer program (in the website of BioInformatics & Molecular Analysis Section (BIMAS) HLA Peptide Binding Predictions). | | | | |

### [Example 2] Identification of adenovirus-specific CTL epitope peptide - HLA type screening

Candidate peptides for adenovirus-specific CTL epitopes have an HLA-A*24 molecule-binding motif. Thus, it is preferred that the epitope peptides are identified using peripheral blood derived from persons possessing the HLA-A*24 molecule. Accordingly, a serum test was first performed to determine whether the blood donors possessed the HLA-A*24 or HLA-A*2 molecule. Specifically, an anti-HLA-A*24 antibody (clone name: 22E1, MBL Co.) or anti-HLA-A*2 antibody (clone name: BB7.2, MBL Co.) was added at the concentration of 1 or 10 µg/ml to 100 µl of peripheral blood from healthy adults or 2 to 10 x 10⁵ PBMCs isolated from peripheral blood. An isotype antibody for each was similarly added as a negative control. The reaction was conducted at room temperature for 15 to 30 minutes, and then the samples were reacted with a fluorescein isothiocyanate (FITC)-labeled anti-mouse IgG antibody. When peripheral blood was used, the blood was treated according to a method using an erythrolytic/fixing reagent (OptiLyse B, Beckman Coulter). PBMCs were washed. Then, their reactivity was examined by flow cytometer FACSCalibur (Becton Dickinson). Examples of the determination of HLA types are shown in Fig. 1. Subsequent examinations were carried out using PBMCs of ten healthy adults that were reactive to the anti-HLA-A*24 antibody.

### [Example 3] Identification of adenovirus-specific CTL epitope peptide - Preparation of antigen-presenting cells

### (1) Preparation of EBV-infected B cell line

PBMCs were co-cultured with a supernatant (containing live EBV) of B95-8 cells (obtained from JCRB Cell Bank), an EBV-producing cell line, according to a conventional method (Kuzushima K, Yamamoto M, Kimura H, Ando Y, Kudo T, Tsuge I, Morishima T. Establishment of anti-Epstein-Barr virus (EBV) cellular immunity by adoptive transfer of virus-specific cytotoxic T lymphocytes from an HLA-matched sibling to a patient with severe chronic active EBV infection. Clin Exp Immunol. 103:192-198 (1996)) to establish an EBV-infected B cell line (lymphoblastoid cell line; hereinafter referred to as EBV-infected LCL). After about two weeks, the expression of HLA molecule, CD80, CD83, and CD86 was confirmed.

### (2) Preparation of CD40-B cells

NIH3T3 cells (NIH-CD40L) in which the human CD40L gene has been introduced and stably expressed were co-cultured with PBMCs in the presence of IL-4. 96 Gy of X-ray was irradiated to inhibit the growth of NIH-CD40L and the co-culture was repeated every three to four days (Kondo E, Topp MS, Kiem HP, Obata Y, Morishima Y, Kuzushima K, Tanimoto M, Harada M, Takahashi T, Akatsuka Y. Efficient generation of antigen-specific cytotoxic T cells using retrovirally transduced CD40-activated B cells. J Immunol. 169:2164-2171 (2002)). The expression of HLA molecule, CD80, CD83, and CD86 was confirmed after about two weeks.

### (3) Preparation of dendritic cells

PBMCs were cultured in a plastic culture dish in a 5% CO₂ incubator at 37°C for two hours. Then, cells that had not adhered to the dish were removed by lightly washing. GM-CSF and IL-4 were added to the dish, and the cells were cultured for 24 hours. Then, TNFα, IL-1β, and PGE2 (Prostaglandin E2) were added, and the cells were cultured for 24 to 48 hours. The dish was lightly washed with an appropriate culture medium or such, and the collected cells were taken as dendritic cells (Dauer M, Obermaier B, Herten J, Haerle C, Pohl K, Rothenfusser S, Schnurr M, Endres S, Eigler A. Mature dendritic cells derived from human monocytes within 48 hours: a novel strategy for dendritic cell differentiation from blood precursors. J Immunol. 170:4069-4076 (2003)). The expression of HLA molecule, CD80, CD83, and CD86 was confirmed after 48 hours.

### [Example 4] Identification of adenovirus-specific CTL epitope peptide - Induction of adenovirus-specific CTLs

### (1) Induction using antigen-presenting cells

The above-described antigen-presenting cells (EBV-infected LCL, CD40-B cells, and dendritic cells) were previously prepared from PBMCs of healthy adults that were reactive to the anti-HLA-A*24 antibody. The antigen-presenting cells were suspended in a pulsing medium (0.1% human serum albumin/55 µM 2-mercaptoethanol/RPMI 1640) or AIM-V medium (Invitrogen), and a candidate CTL epitope peptide was added at a concentration of 10 µg/ml. The resulting mixture was allowed to stand at room temperature for 30 to 60 minutes while mixing gently in intervals of about 15 minutes. Then, the cells were washed three times with an excess amount of washing solution (2% fetal calf serum (FCS)/PBS) to remove peptides that were not bound to the HLA molecule. This treatment is assumed to allow a candidate CTL epitope peptide to bind to HLA molecules on antigen-presenting cells. Antigen-presenting cells treated as described above are called peptide-pulsed antigen-presenting cells. The peptide-pulsed antigen-presenting cells were treated with a lethal dose of X-ray irradiation or mitomycin C to eliminate the growth ability. The cells were combined with PBMCs, or CD8-positive or CD4-positive T cells isolated from the same persons, and were cultured in a 5% CO₂ incubator at 37°C. Regarding the medium used, RPMI1640 containing 10% FCS, RPMI1640 containing 10% human serum, RPMI1640 containing 1 to 10% human plasma, and others were tested, and the RPMI1640 containing 10% human serum gave a favorable result in this method. IL-2 (Shionogi & Co., Ltd.) was added to maintain the viability of T cells and support their growth. The timing of addition is typically after 7 to 10 days of the start of mix culture in many reports. However, when the timing was tested in the present invention by adding IL-2 at the start of culture, after two days and six days of the start of culture, and other cases, a favorable result was obtained when it was added two days after the start of culture. Thus, IL-2 was added at 50 U/ml two days after the start of culture. The cells were stimulated again using peptide-pulsed antigen-presenting cells seven days after the start of culture. The cells were stimulated every week using peptide-pulsed antigen-presenting cells. The induction of CTLs was evaluated after about two and four weeks. When the induction of adenovirus-specific CTLs was confirmed, the cells were further stimulated with peptide-pulsed antigen-presenting cells to establish CTL lines.

### (2) Induction method without using antigen-presenting cells

This induction method induces CTLs by adding peptides to culture media of PBMCs. Peptides are presented on antigen-presenting cells present among PBMCs, such as dendritic cells, B cells, macrophages, and certain types of T cells, whereby CTL precursor cells in PBMCs are stimulated to proliferate. Unlike the above-described induction method which utilizes antigen-presenting cells, this method does not require the prior preparation of antigen-presenting cells and is therefore advantageous in its ease of application.

Peripheral blood collected from healthy adults that were reactive to the anti-HLA-A*24 antibody was centrifuged at 3,000 rpm for 5 to 10 minutes. The resulting supernatant plasma was collected. The non-plasma portion was processed according to a conventional method to isolate PBMCs. The characteristic of the present method is the addition of several % of plasma to an induction medium. In the present invention, a favorable result was obtained by adding 5% plasma. The medium is prepared by adding appropriate additives and antibiotics to a medium commonly used for cell culture. The CTL induction medium used in the present invention was RPMI1640 Hepes modify (Sigam) supplemented with 2-mercapotethanol, L-glutamine, and the antibiotics streptomycin and penicillin. In addition to these, insulin, transferrin, selenious acid, pyruvic acid, human serum albumin, solution of non-essential amino acids, and the like may be added. 1 to 3 x 10⁶ PBMCs were suspended in 1 to 2.5 ml of the culture medium. A candidate peptide was added to the cells at a concentration of 1 to 20 µg/ml. The concentration of peptide can be altered depending on the solubility of the peptide. The peptide was added at 10 µg/ml. After two days, IL-2 was added at a final concentration of 20 to 100 U/ml. Round-bottomed culture dishes that allow the exchange of carbon dioxide gas are preferably used to co-culture PBMCs with peptides. In the context of the present invention, 14-ml round-bottomed polypropylene tubes (Becton Dikinson) or 96-well U-bottomed cell culture microtest plates (Becton Dikinson) were used. The adenovirus-specific CTLs were confirmed after about two and four weeks of culture. The cells were stimulated again with the peptide at 10 µg/ml after about two weeks of culture. When the induction of adenovirus-specific CTLs was confirmed, the cells were further stimulated with peptide-pulsed antigen-presenting cells to establish CTL lines.

### [Example 5] Identification of adenovirus-specific CTL epitope peptide - Confirmation of the adenovirus-specific CTLs

The presence of adenovirus-specific CTLs in the groups of cells cultured by the methods described above was tested by the method for quantifying cells producing intracellular IFNγ, MHC-tetramer method, or ELISPOT assay, or expression test of membrane surface proteins.

### 1. Confirmation of induction of specific CTLs by the method for quantifying cells producing intracellular IFNγ

Approximately 1/10 to all of the cells induced by the above-described method were transferred into 96-well U-bottomed cell culture microtest plates, and the peptide used for the induction was added thereto at a final concentration of 0.01 to 0.05 µg/ml. Then, an intracellular protein transport inhibitor (for example, brefeldin A, monensin, or such) was added, and the cells were cultured in a 5% CO₂ incubator at 37°C for 5 to 16 hours. After culture, the cells were washed, and phycoerythrin (PE)-labeled MHC-tetramer reagent and phycoerythrin-Cy5(PCS)-labeled CD8 antibody (Beckman Coulter) were added thereto. The resulting mixture was allowed to stand at room temperature for 15 to 30 minutes. After washing, the cells were fixed with 4% formaldehyde at 4°C for 15 minutes and washed with an excess amount of washing solution. After membrane permeabilization treatment with 0.1 % saponin, an FITC-labeled anti-IFNγ antibody (Beckman Coulter) was added, and reacted at room temperature for 15 to 30 minutes. After washing, the proportion of IFNγ-positive cells in T cells or MHC-tetramer reagent-positive cells was determined using a flow cytometer.

Fig. 2 shows the results obtained by inducing specific CTLs using known positive control peptides by the induction method without antigen-presenting cells and assaying the cells by the method for quantifying cells producing intracellular IFNγ. PBMCs from donor ID *24-2 were stimulated with the HLA-A*2402-restricted epitope peptide of EBV BRLF1 (a and b) or CMV pp65 (c and d) for 13 days, and then stimulated in the presence of monensin for 14 hours with the negative control (HIV) peptide (a and c) and each peptide (b and d) used for the stimulation. The cells were triple-stained with PE-labeled MHC-tetramer reagent (MBL Co.), PC5-labeled CD8 antibody, and FITC-labeled IFNγ antibody. The results obtained by analyzing the cells with a flow cytometer is shown. The numerals in the dot plots indicate the proportion (%) of cells in a quarter area to the whole viable cells. Hereinafter the quarter areas are referred to as UL (upper left), UR (upper right), LL (lower left), and LR (lower right). In the dot plots (in the left column) where the X- and Y-axes indicate the fluorescence intensities for CD8 and INFγ in the log scale, IFNγ-positive CD8-positive cells appear in UR only upon restimulation with each of the specific peptides of EBV BRLF1 and CMV pp65 (b and d), while such cells hardly appear upon addition of the HIV peptide (a and c). However, the presence of EBV BRLF1-specific and CMV pp65-specific CTLs in the cell populations to which the HIV peptide or positive control peptide was added is evident from the CD8-positive MHC-tetramer reagent-positive cells in UR of the dot plots (in the middle column) where the X- and Y-axes indicate the fluorescence intensities for CD8 and the MHC-tetramer reagent in the log scale. Specifically, it was found that when the HIV peptide was added, EBV BRLF1-specific and CMV pp65-specific CTLs accounted for 13.0% and 28.8%, respectively, but the CTLs produced no IFNγ without specific peptide stimulation. Furthermore, in the dot plots (in the right column) where the X- and Y-axes indicate the fluorescence intensities for IFNγ and MHC-tetramer reagent, most of the MHC-tetramer reagent-positive cells (UL and UR) were present in UL, producing no IFNγ, when the HIV peptide were added (a and c). However, upon stimulation with a specific peptide (b and d), more than half of the cells shifted to UR, producing IFNγ.

These results demonstrate the restimulation induced IFNγ-producing cells from PBMCs cultured in the presence of a CTL epitope peptide; and that the cells were specific CTLs because they were stained with the MHC-tetramer reagent. Accordingly, whether specific CTLs were induced could be determined by the method for quantifying cells producing intracellular IFNγ. Similarly, whether specific CTLs were induced using candidate peptides for adenovirus-specific CTL epitopes was examined using PBMCs of ten healthy adults. Fig. 3a-d and Fig. 3e-h show the results of quantification of intracellular IFNγ-producing cells in representative two persons (donor ID*24-8 and *24-12).

With EBV BRLF1, a positive control, a large number of CD8-positive, IFNγ-positive (producing) cells are seen in UR (24.9% for *24-8; and 9.57% for *24-12). In contrast, almost no positive cells appear in UR when the HIV peptides were added for restimulation as a negative control (0.62% for *24-8; 0.18% for *24-12). The results of comparing the ratios of CD8-positive, IFNγ-positive cells (UR) in the cases of addition of the HIV peptide and restimulation with the candidate peptides for adenovirus-specific CTL epitope confirmed that VYS, LYA, TYF, and GYK induced specific CTLs in donor ID *24-8 (Fig. 3a-d). Although LYA showed no difference as compared to when the HIV peptide was added, , IFNγ-producing cells with strong fluorescence intensity were observed as dots in UR when the LYA peptide was used for restimulation, suggesting the induction of specific CTLs. Likewise, specific CTL induction was confirmed in donor ID *24-12 (Fig. 3e-h) when DYL or LYS was used.

### 2. Confirmation of CTLs by ELISPOT assay

A commercially available ELISPOT assay kit (for example, MABTECH Co.) may be purchased, and the assay can be performed according to the manufacturer's protocol. Alternatively, the assay may also be carried out by the following procedure. 96-well MultiScreen-HA plates (Millipore Co.) were coated with an anti-IFNγ monoclonal antibody (R&D Systems Co.) at 4°C for overnight. Each well was washed with PBS. Target cells were plated into each well. Any cells expressing HLA-A*24 molecules on their surface can be used as the target cells. For example, T2-A24, a cultured cell which a gene of HLA-A*24 molecule is introduced into a 174CEM.T2 cell, can be used. Alternatively, EBV-infected LCLs expressing HLA-A*24 molecules on their surface can be used as the target cells. These target cells were added into each well with a candidate peptide for adenovirus-specific CTL epitopes or a negative control (HIV) peptide. The mixtures were allowed to stand at room temperature for 30 minutes. Then, an adequate number of CTLs were added, and the mixtures were cultured in a 5% CO₂ incubator at 37°C for 20 hours. CTLs that responded to the candidate epitope peptide secreted IFNγ during this culture period, and the IFNγ bound to the anti-IFNγ monoclonal antibody that was coated on the plates. After culturing was completed, the plates were washed with PBS containing 0.05% Tween-20. Then, an anti-IFNγ polyclonal antibody (Genzyme Co.) and peroxidase-labeled goat anti-rabbit IgG serum were allowed to react in this order at room temperature for 90 minutes each. Furthermore, 3-amino-9-ethylcarbasole (Sigma), a peroxidase substrate, and 0.1M sodium acetate buffer (pH 5.0) containing 0.015% H₂O₂ was added to each well, and the plates were incubated at room temperature for 40 minutes to visualize the IFNγ spots. The spots were counted under a stereomicroscope.

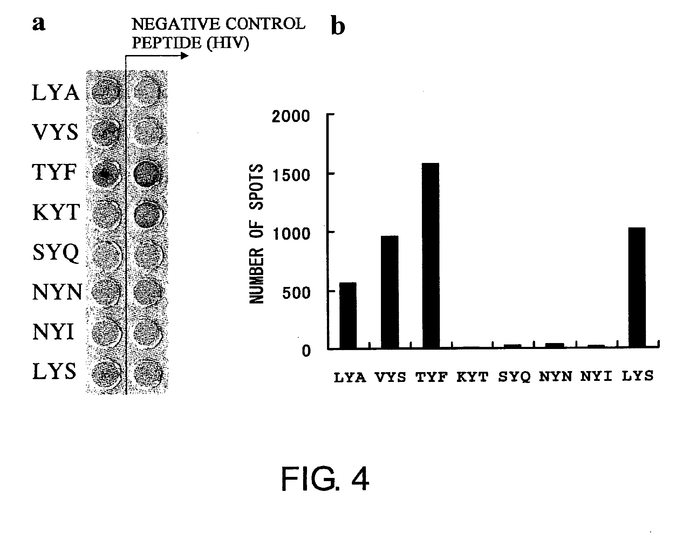

Fig 4 shows an example of detecting specific CTLs by ELISPOT assay. IFNγ spots were observed by comparing the candidate peptides for adenovirus-specific CTL epitopes, LYA, VYS, TYF; and LYS, with the negative control (HIV) peptide. The spots were counted under a stereomicroscope, and were plotted on a graph.

### 3. Confirmation of CTLs based on membrane surface protein expressions

It has been reported that CTL, upon specific stimulation, show increased expression of cell surface proteins (for example, CD107a, CD 1 07b, CD63, CD69, and the like). Approximately 1/10 to all of the cells induced by the above-described method was transferred into 96-well U-bottomed cell culture microtest plates, and peptides used for the induction was added thereto at a final concentration of 0.01 to 0.05 µg/ml. Then, the cells were cultured in a 5% CO₂ incubator at 37°C for 5 to 16 hours. After culturing was completed, the cells were washed, each variously labeled antibody against a cell surface protein and the like, and MHC-tetramer reagent or CD8 antibody were added thereto. After gentle stirring, the cells were allowed to stand at room temperature for 15 to 30 minutes. Then, the cells were washed, and the number of cells expressing various membrane proteins from among the CD8-positive cells or MHC-tetramer-positive cells was examined using a flow cytometer.

The results of evaluating candidate epitope peptides using the cytokine quantification method are shown in Table 2. In the Table, "open circle" indicates that the use of a candidate peptide enabled specific CTLs to be induced, and "cross" indicates that specific CTLs was unable to be induced. PBMCs derived from ten people with HLA-A*24 molecule were tested using 10 types of peptides. As a result, six types of candidate epitope peptides DYL, VYS, LYA, TYF, LYS, and GYK were found to induce cytokine production in a peptide-specific manner. Thus, these six types of candidate epitope peptides serve as adenovirus-specific CTL epitope peptides. Since the adenovirus-specific CTL epitope peptides have the HLA-A*24-binding motif, they are likely to be HLA-A*2402-restricted epitope peptides, which are carried by 60% or more of the Japanese people. In the context of the results described above, MHC-tetramer reagents were synthesized using five types of epitope peptides, and their utility was tested.

**[Table 2]**

| DONOR ID | EBV BRLF1 | CANDIDATE PEPTIDES FOR ADENOVIRUS TYPE 11 HEXON EPITOPE | | | | | | | | | |
|---|---|---|---|---|---|---|---|---|---|---|---|
| | | KYT | DYL | VYS | LYA | TYF | SYQ | LYS | NYN | NYI | GYK |
| *24-1 | ○ | × | ○ | × | × | × | × | × | × | × | × |
| *24-2 | ○ | × | ○ | ○ | × | ○ | × | × | × | × | × |
| *24-3 | ○ | × | × | × | ○ | × | × | × | × | × | × |
| *24-5 | × | × | × | × | × | × | × | × | × | × | × |
| *24-8 | ○ | × | × | ○ | ○ | ○ | × | × | × | × | ○ |
| *24-10 | ○ | × | ○ | × | × | × | × | × | × | × | × |
| *24-11 | ○ | × | × | × | × | × | × | × | × | × | × |
| *24-12 | ○ | × | ○ | × | × | × | × | ○ | × | × | × |
| *24-13 | ○ | × | × | × | × | × | × | ○ | × | × | × |
| *24-14 | ○ | × | × | × | × | × | × | × | × | × | × |

### [Example 6] Examination using adenovirus-specific MHC-tetramer reagents

### [Synthesis of MHC-tetramer reagents]

MBL Co. where the present inventors are affiliated with has acquired exclusive patent licensing rights in Japan for MHC-tetramer reagents (U.S. Patent No. 5,635,363; French Application No. FR9911133; and U.S. Patent Nos. 5,723,584, 5,874,239, 5,932,433, and 6,265,552) from Beckman Coulter Co. Based on the patents, PE-labeled MHC-tetramer reagents were prepared using adenovirus-specific CTL epitope peptides and HLA-A*2402 molecules. The MHC-tetramer reagents prepared in the context of the present invention are abbreviated as TYF-Tet, for example, indicating that the reagent was synthesized using the ternary complex of HLA-A*2402 molecule, peptide TYF (TYFNLGNKF/ SEQ ID NO: 4), and β2-microglobulin.

### [Quantification method using MHC-tetramer reagents]

Adenovirus-specific CTLs quantification was carried out using MHC-tetramer reagents that was prepared using CTL epitope peptides of the present invention, with peripheral blood, PBMCs isolated from peripheral blood, CTL lines induced using CTL epitope peptides as samples. Examples of quantification using PE-labeled MHC-tetramer reagents are described below. Labelling dyes may be used in an appropriate combination depending on the type of flow cytometer to be used, and is not limited to the examples described below.

### <Using peripheral blood>

Ten µl of a PE-labeled MHC-tetramer reagent, 20 µl of an FITC-labeled antibody for T cell (for example, CD8, CD4, or CD3), and such were added to 200 µl of collected peripheral blood. A CD45 antibody labeled with PC5 or such may also be added to eliminate nonspecific fluorescence due to contaminated erythrocytes. After gentle mixing, the mixture was allowed to stand at room temperature for 30 minutes. OptiLyse B was added, and erythrolysis and fixation was carried out according to the manufacturer's protocol. Two ml of PBS was added, stirred, and the cells were centrifuged at 400 x g for 5 minutes. After the supernatant was removed by aspiration, the cells were resuspended in 500 µl of PBS and analyzed using a flow cytometer within 24 hours.

### <Using PBMCs or CTL lines induced by use of CTL epitope peptides >

Ten µl of a PE-labeled MHC-tetramer reagent, 20 µl of an FITC-labeled T cell surface antibody (for example, CD8, CD4, or CD3), and such were added to an appropriate amount of PBMCs (10⁵ to 10⁶ cells) or a CTL line induced by use of CTL epitope peptides. A CD45 antibody labeled with PC5 or such may also be added to eliminate nonspecific fluorescence due to contaminated erythrocytes. After gentle mixing, the cells were allowed to stand at room temperature for 30 minutes. Three ml of PBS was added, stirred, and the cells were centrifuged at 400 x g for 5 minutes. After the supernatant was removed by aspiration, the cells were resuspended in 500 µl of PBS. When a CTL line is used, 7-AAD Viability Dye (Non-viable cell detection reagent; MBL Co.) may be added to eliminate nonspecific fluorescence caused by non-viable cells. The cells were analyzed using a flow cytometer within 24 hours.

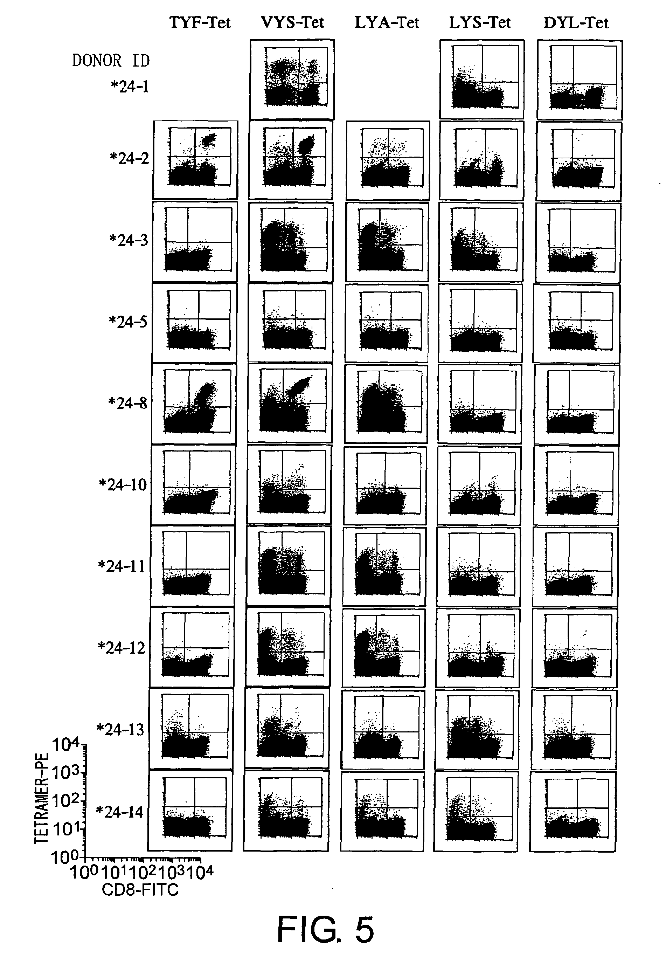

The results of staining obtained using the five types of MHC-tetramer reagents used to prepare the peptide-induced CTL lines are shown in Fig. 5. The results indicate by dot plots where the X- and Y-axes indicate fluorescence intensity for CD8 and MHC-tetramer reagent respectively, in log scale.

### (1) Reactivity of HLA-A*2402 TYFNLGNKF (SEQ ID NO: 4) tetramer reagent (TYF-Tet)

Regarding TYF-Tet, a population of CD8-positive, TYF-Tet-positive cells was clearly detected in the UR for donor ID*24-2 and *24-8. This demonstrates that TYF is an HLA-A*2402-restricted adenovirus hexon-specific CTL epitope peptide.

### (2) Reactivity of HLA-A*2402 VYSGSIPYL (SEQ ID NO: 3) tetramer reagent (VYS-Tet)

Regarding VYS-Tet, a population of CD8-positive, VYS-Tet-positive cells was clearly detected in the UR for donor ID*24-2 and *24-8. This demonstrates that VYS is an HLA-A*2402-restricted adenovirus hexon-specific CTL epitope peptide. Alternatively, regarding VYS-Tet, a population of CD8-negative, VYS-Tet-positive cells was detected in the UL for donor ID *24-1, *24-3, *24-11, *24-12, and *24-14. This suggests that VYS is an epitope peptide capable of inducing not only the CD8-positive CTLs but also the CD8-negative's.

### (3) Reactivity of HLA-A*2402 LYANSAHAL (SEQ ID NO: 2) tetramer reagent (LYA-Tet)

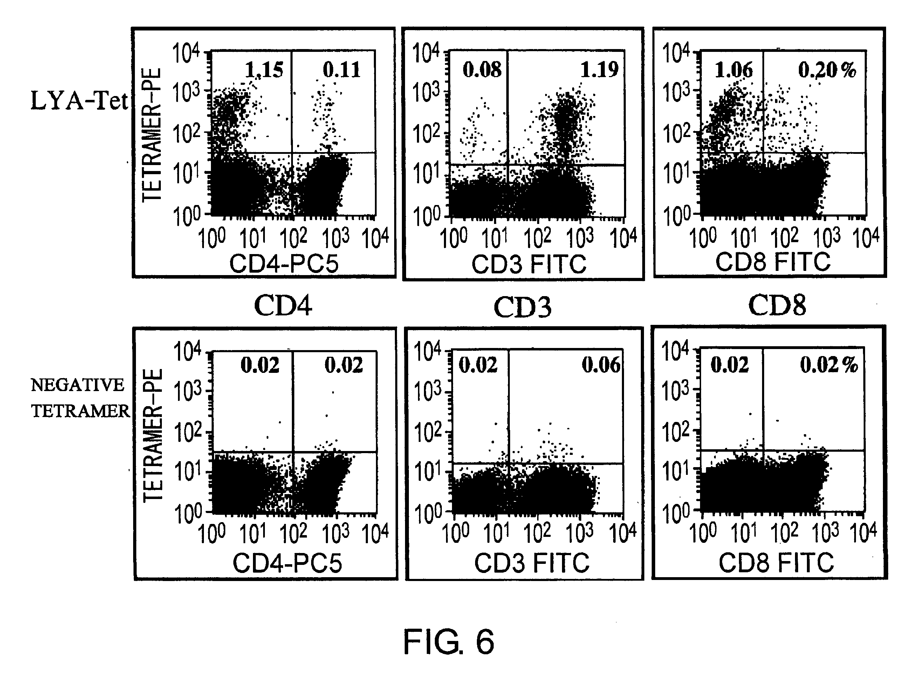

Regarding LYA-Tet, a population of CD8-negative, tetramer reagent-positive cells was detected in the UL. This suggests the presence of CD8-negative, LYA-Tet-positive CTLs. Results of a further detailed examination are shown in Fig. 6. After 24 days of stimulation of PBMCs from donor ID*24-14 with LYA, the cells were stained with LYA-Tet, and CD4, CD3, or CD8. The reactivity was compared to that of the Negative Tetramer reagent (MBL Co.) used as a negative control. The results demonstrate that CD4-positive, LYA-Tet-positive cells accounted for 0.09%, CD8-positive, LYA-Tet-positive cells accounted for 0.18%, and the proportion of CD3-positive, LYA-Tet-positive cells (1.13%) accounted for the most. This suggests the possibility that CD4-negative, CD8-negative, CD3-positive HLA class I-restricted CTLs exist and they are involved in elimination of adenovirus-infected cells.

### (4) Reactivities of HLA-A*2402 LYSNVALYL (SEQ ID NO: 5) tetramer reagent (LYS-Tet) and HLA-A*2402 DYLSAANML (SEQ ID NO: 1) tetramer reagent (DYL-Tet)

As shown in Table 2, LYS was shown to result in the production of IFNγ in a peptide-specific manner in two of the ten persons tested. As shown in Fig. 3e-h, no relevant profile of LYS-Tet-positive cells was obtained for donor ID *24-12 although IFNγ-producing cells accounted for as much as 2.95% when LYS was used. Alternatively, as shown in Table 2, DYL was found to result in the production of IFNγ in a peptide-specific manner in four of the ten persons tested. As shown in Fig. 3e-h, no relevant profile of DYL-Tet-positive cells was obtained although IFNγ-producing cells accounted for as much as 0.49% when DYL was used. These results strongly suggest that LYS and DYL are not restricted by HLA-A*2402 but by other HLA molecules. HLA restriction of LYS can be clarified by analyzing HLA genotypes of the two persons, donor ID*24-12 and *24-13, and searching for a common HLA other than HLA-A*24 in these two persons. HLA restriction of DYL can be clarified by analyzing HLA genotypes of the four persons, donor ID*24-1, *24-2, *24-10, and *24-12, and searching for a common HLA other than HLA-A*24 in these four persons. A recent research paper has reported that QYDPVAALF (SEQ ID NO: 12; amino acid No. 341-349), an HLA-A*2402-restricted epitope peptide of HCMV pp65 protein, is also restricted by HLA-Cw*0401 (Kondo E, Akatsuka Y, Kuzushima K, Tsujimura K, Asakura S, Tajima K, Kagami Y, Kodera Y, Tanimoto M, Morishima Y, Takahashi T. Identification of novel CTL epitopes of CMV pp65 presented by a variety of HLA alleles. Blood. 103:630-638 (2004)). In an HLA-A*24-restricted peptide, Tyr, Phe, Met, or Trp is arranged at the second position from the N terminus, and Leu, Ile, Trp, or Phe is arranged at position 9 or 10. In an HLA-Cw*0401-restricted peptide, Tyr, Phe, or Pro is arranged at the second position from the N terminus, and Leu or Phe is arranged at position 9 or 10. Thus, the two peptides are quite alike. The actual scores, calculated by BIMAS, were 280 for A*24 and 200 for Cw*0401 regarding LYS. Likewise, regarding DYL, the scores were 360 and 220 for A*24 and Cw*0401, respectively. In a same manner, scores for LYS and DYL associated with HLA-A, -B, and -C whose frequencies are high in the Japanese people were sought. However, no other HLA beside HLA-Cw*0401 had such a high score. These result suggest the possibility of HLA-Cw*0401 being responsible for the HLA restriction of LYS and DYL.

### [Example 7] Detection of adenovirus-specific CTLs in peripheral blood of healthy adults using MHC-tetramer reagents

It is important to know whether adenovirus-specific CTLs are present in peripheral blood of transplantation donors or other high-risk patients, such as immunocompromised patients of any cause, patients having a congenital immunodeficiency, patients who received an immunosuppressant to prevent rejection after transplantation of bone marrow, hematopoietic stem cells, cord blood, or a solid organ, patients with chronic virus infection, AIDS patients, elderly people, babies and infants, and pregnant women. Such information is vital for infection management including the determination of the appropriate use of anti-viral agents and immunosuppressants. When adenovirus-specific MHC-tetramer reagents are used, the presence of adenovirus-specific CTLs can be determined within about an hour later the blood collection.

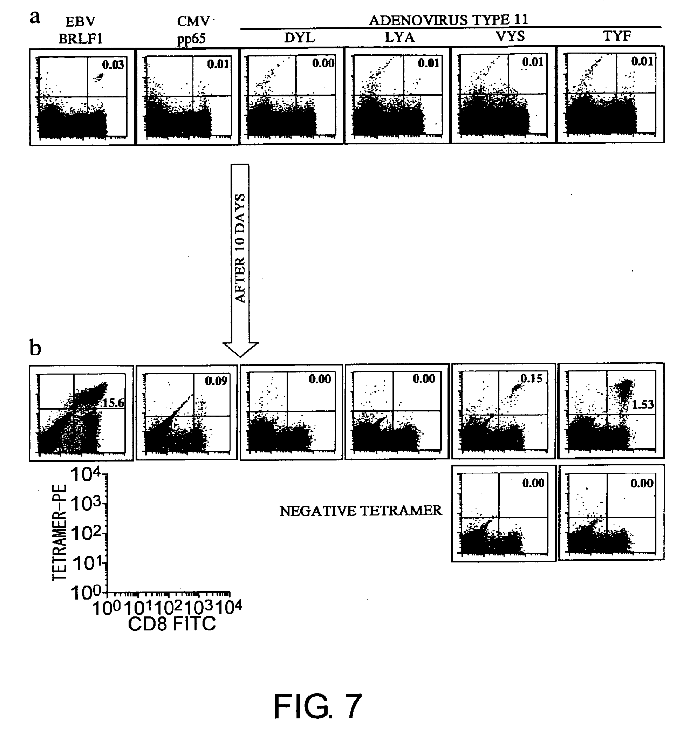

Accordingly, the present inventors examined whether adenovirus-specific CTLs could be detected by using peripheral blood from healthy adults. The results are shown in Fig. 7a. Staining was carried out using peripheral blood of donor ID*24-8 and four types of adenovirus-specific MHC-tetramer reagents, and EBV BRLF1 and CMV pp65 MHC-tetramer reagents (MBL Co.). The results indicate by dot plots where X- and Y-axes indicate fluorescence intensity for CD8 and INFγ respectively, in log scale. The numeral in each dot plot indicates a positive rate (%) that is the proportion of CD8-positive, MHC-tetramer reagent-positive cells to viable cells.

As a result, a population of CD8-positive, MHC-tetramer reagent-positive cells was only clearly detected for EBV BRLF1 (UR: 0.03%) immediately after blood collection (day 0). Likewise, no clearly positive cell population was detected by using the adenovirus-specific MHC-tetramer reagent in the other nine persons. It is possible that all the peripheral blood used was derived from healthy adults who were unlikely to be infected with adenovirus or to develop an adenovirus infection.

In contrast, 90% of adults are infected with EBV. Thus, as shown in Table 2, the induction of EBV BRLF1-specific CTLs succeeded in nine of ten persons examined in the present invention. Accordingly, it is highly possible to be able to easily detect a subject who is undoubtedly infected or likely to be infected with an adenovirus even at day 0. Then, PBMCs were cultured stimulatively with each epitope peptide for 10 days (day 10) and were stained with the MHC-tetramer reagents in the same way as above (Fig. 7b).

The results showed that, in the case of EBV BRLF1, ten days of culture increased the positive rate of about 520 times, from 0.03 to 15.6%. Each of TYF-Tet and VYS-Tet gave a clear CD8-positive, MHC-tetramer-positive pattern as compared to Negative Tetramer reagent (MBL Co.). It was observed that TYF and VYS increased the positive rate of adenovirus-specific CTLs by 153 times (0.01 to 1.53%) and 15 times (0.01 to 0.15%), respectively.

These results revealed that as compared to the EBV-specific CTLs, the positive rate of adenovirus-specific CTLs is low but they are present in the peripheral blood of healthy adults. Moreover, it was revealed that after 10 days of culturing, the adenovirus-specific CTLs can be proliferated to a level that allows determination of the presence of CTLs. This means that the induction method of the present invention that does not require the use of antigen-presenting cells is convenient for detecting adenovirus-specific CTLs as well as are effective for a short-term culturing of CTLs.

### [Example 8] Assessment of CTL functionality using MHC-tetramer reagents

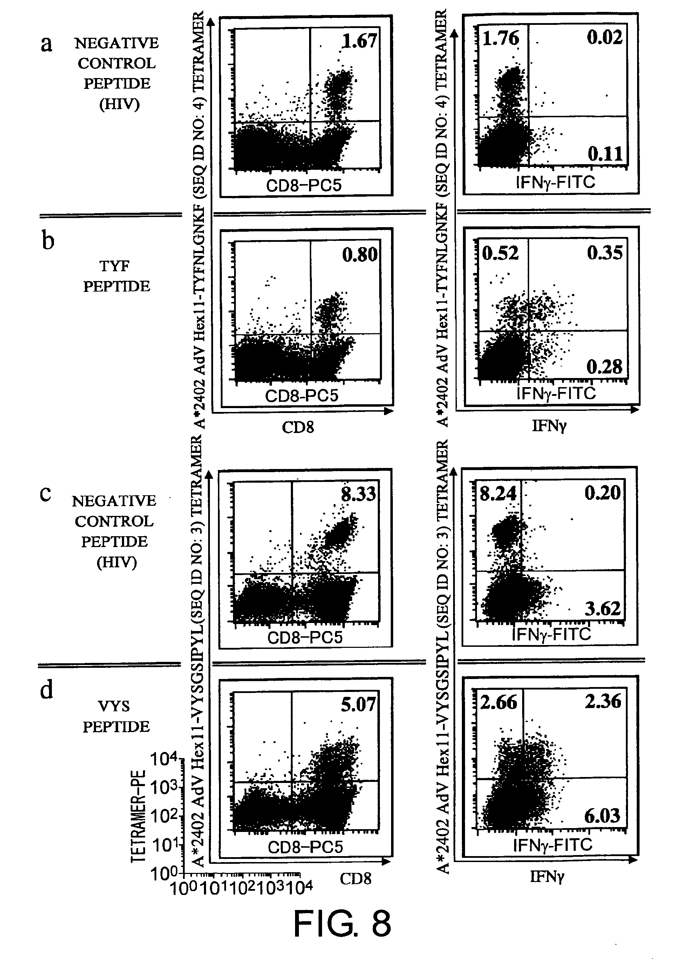

Not only specific CTLs can be quantified but also their differentiation stages and functionality can be assessed at the same time by using the MHC-tetramer reagents in combination with methods for quantifying cells producing intracellular cytokines or with specific antibodies against cell surface proteins. MHC-tetramer reagents synthesized by using CTL epitope peptides provided by the present invention were examined whether they are effective to quantify adenovirus-specific CTLs and to analyze their functions. The results are shown in Fig. 8.

Fig. 8a and b depict the results obtained by analyzing PBMCs from donor ID*24-8 with the method for quantifying cells producing intracellular IFNγ after 10 days of stimulation of cells with TYF peptides. Fig. 8c and d depict the results obtained by analyzing PBMCs from donor ID*24-2 with the method for quantifying cells producing intracellular IFNγ after 27 days of stimulation of cells with VYS peptides. Fig. 8a and c depict the results of restimulation with the negative control (HIV) peptide; Fig. 8b and d depict the results of restimulation with the specific peptides. In the left, the results of CTL quantification are indicated by dot plots where the X- and Y-axes indicate the fluorescence intensities for CD8 and the MHC-tetramer reagent in the log scale, respectively. In the right, the qualitative results for the CTLs are indicated by dot plots where the X- and Y-axes indicate the fluorescence intensities for IFNγ and the MHC-tetramer reagent in the log scale, respectively. The numeral in each dot plot indicates the proportion (%) of cells in a quarter area to the whole viable cells.

The results of quantitation of specific CTLs demonstrate that the amount of specific CTLs was reduced by the stimulation with the specific peptides as compared to the case of stimulation with the HIV peptide; 1.67% to 0.80% for TYF-specific CTLs and 8.33% to 5.07% for VYS-specific CTLs.

The reason is that the expression level of TCR on the cell surface of specific CTLs is reduced upon specific stimulation (Valitutti S, Muller S, Dessing M, Lanzavecchia A. Different responses are elicited in cytotoxic T lymphocytes by different levels of T cell receptor occupancy. J Exp Med. 183:1917-1921 (1996); Betts MR, Price DA, Brenchley JM, Lore K, Guenaga FJ, Smed-Sorensen A, Ambrozak DR, Migueles SA, Connors M, Roederer M, Douek DC, Koup RA. The functional profile of primary human antiviral CD8+ T cell effector activity is dictated by cognate peptide concentration. J Immunol. 172:6407-6417 (2004)). The cell population in UR shifted toward the X-axis upon addition of the specific peptides, compared to when the HIV peptide was added. This also clearly indicates that the reactivity to the MHC-tetramer reagent, namely, the expression level of TCR that binds to the MHC-tetramer reagent, was specifically reduced. In other words, this indicates the existence of adenovirus-specific CTLs. The qualitative results for the specific CTLs demonstrated that there were almost no TYF-specific CTLs (1.76%) nor VYS-specific CTLs (8.24%) producing IFNγ among MHC-tetramer reagent-positive cells (UL and UR) when the HIV peptide was added. However, the cell population was clearly demonstrated to shift to UR or LR, and to produce IFNγ upon stimulation with the specific peptides.

These results demonstrate that adenovirus-specific CTLs producing IFNγ can be induced efficiently by *ex vivo* peptide stimulation.

### [Example 9] Purification of CTLs using MHC-tetramer reagents

A PE-labeled adenovirus-specific MHC-tetramer reagent was mixed with CTLs induced by the CTL preparation method, and the resulting mixture was allowed to stand at room temperature for 30 minutes. The cells were washed once with an excess amount of washing solution, and magnetically labeled anti-PE microbeads (Miltenyi Biotec GmbH) were added thereto, and the resulting mixture was allowed to stand at 4°C to 8°C for 15 minutes. The cells were washed once with an excess amount of washing solution, and diluted with 1 ml of washing solution. Then, MHC-tetramer-positive cells, namely, adenovirus-specific CTLs were purified using an automatic magnetic cell separation device (AutoMACS, Miltenyi Biotec GmbH).

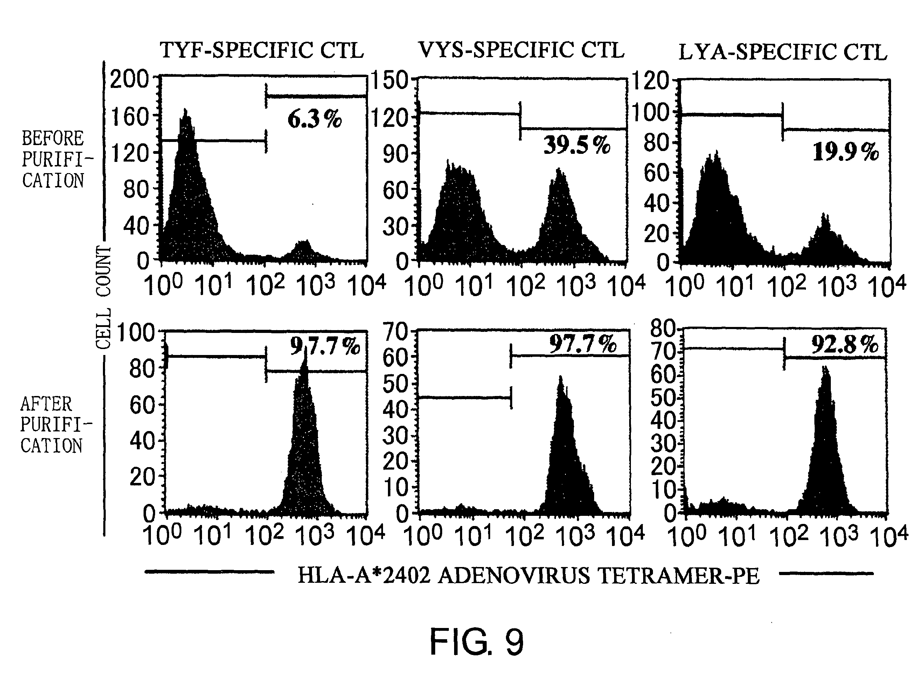

Example of such results are shown in Fig. 9. The results indicate by histograms where the X-axis indicates the fluorescence intensity for the adenovirus-specific MHC-tetramer reagent in the log scale and Y-axis indicates the cell count. The upper and bottom panels indicate analysis results for cell populations that include CTLs before and after purification, respectively. The proportions of specific CTLs in the cell populations before and after purification are shown by numerals. In each case, adenovirus-specific CTLs with 90% or higher purity were collected by this method. This suggests that adenovirus-specific CTLs can be highly purified by using adenovirus-specific MHC-tetramer reagents. The purified adenovirus-specific CTLs were stimulated for growth with an anti-CD3 antibody, IL-2, and antigen-presenting cells whose growth ability had been eliminated by X-ray irradiation to obtain the required number of cells for passive immunotherapy.

### [Example 10] Evaluation of the reactivity between adenovirus subgroups

### [Evaluation of the reactivity of CTL epitope TYF to subgroup C]

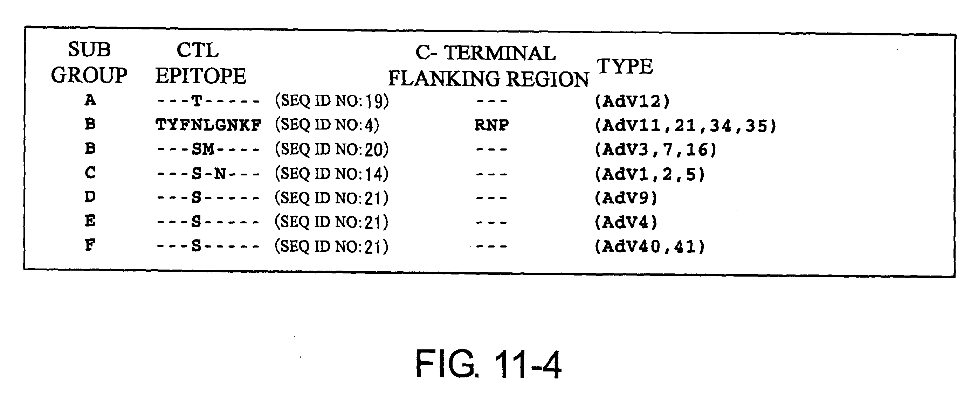

As shown in Fig. 11-4, there are amino acid mutations at the fourth and sixth positions in TYF identified in the present invention as compared to the subgroup C-specific CTL epitopes. Thus, the cross-reactivity to subgroup C-specific CTL epitope (TYFSLNNKF (SEQ ID NO: 14); mutated amino acids are underlined) (Leen AM, Sili U, Vanin EF, Jewell AM, Xie W, Vignali D, Piedra PA, Brenner MK, Rooney CM. Conserved CTL epitopes on the adenovirus hexon protein expand subgroup cross-reactive and subgroup-specific CD8+ T cells. Blood. 104:2432-2440 (2004)) was examined using a CTL line induced by TYF.

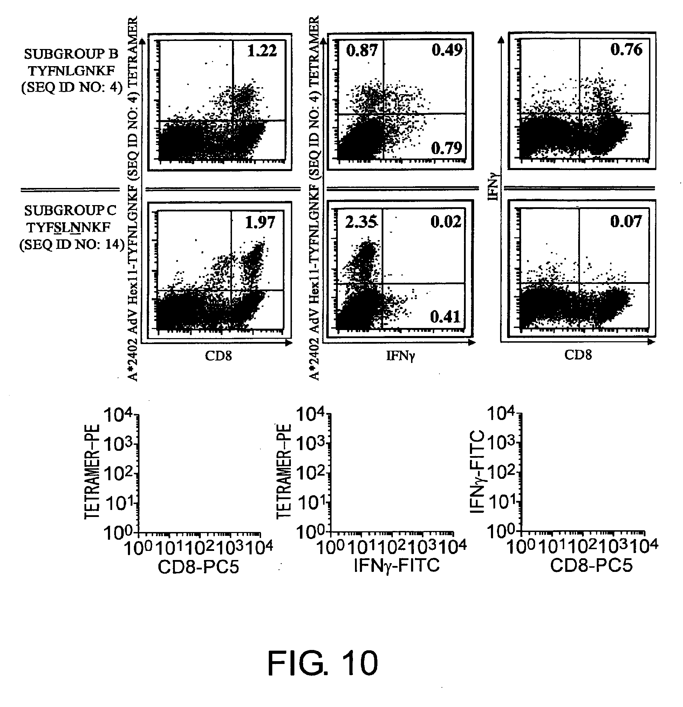

PBMCs prepared from ID*24-8 were stimulated with TYF for 13 days, and then restimulated with TYF or TYFSLNNKF (SEQ ID NO: 14) to examine whether the CTL line produces IFNγ. The results are shown in Fig. 10. The dot plots where the X- and Y-axes indicate the fluorescence intensities for CD8 and TYF-Tet in the log scale, respectively, demonstrate that restimulation with TYF suppressed TCR expression in the TYF-specific CTL line and the number of CD8-positive and TYF-Tet-positive cells was slightly reduced upon addition of TYF (1.22%) as compared to the case where TYFSLNNKF (SEQ ID NO: 14) was added (1.97%). The dot plots where the X- and Y-axes indicate the fluorescence intensities for IFNγ and TYF-Tet in the log scale, respectively, demonstrate that most of TYF-Tet-positive cells (UL and UR) were localized in UL and there was no INFγ-producing cells when TYFSLNNKF (SEQ ID NO: 14) was added while IFNγ-producing cells were observed in UR and LR when TYF was added. The dot plots where the X- and Y-axes indicate the fluorescence intensities for CD8 and IFNγ in the log scale, respectively, demonstrate that many IFNγ-producing cells appeared in UR when TYF was added while almost no such cells were present when TYFSLNNKF (SEQ ID NO: 14) was added. The CTL line induced by TYF produced IFNγ in a TYF-specific manner but did not produce IFNγ in the presence of TYFSLNNKF (SEQ ID NO: 14) whose amino acid sequence differs from TYF at two amino acid positions.

Thus, the TYF CTL epitope peptide identified in the present invention was demonstrated to exhibit no cross-reactivity to subgroup C.

### [Example 11] Assessment of the reactivity of adenovirus hexon proteins by amino acid sequence homology analysis

Fifty types of adenovirus hexon amino acid sequences were obtained from NCBI (National Center for Biotechnology Information, http://www.ncbi.nlm.nih.gov/) (A listing of Accession Numbers is shown in Table 3) and the amino acid sequence homology was analyzed to assess the reactivities of CTL epitope peptides obtained in the present invention between adenovirus subgroups. A recent report describes that mutations of the amino acid immediately after the anchor motif at the C terminus in a CTL epitope alter the sites of proteolysis in the pathway of intracellular proteolysis, resulting in no CTL epitope peptide formation (Beekman NJ, van Veelen PA, van Hall T, Neisig A, Sijts A, Camps M, Kloetzel PM, Neefjes JJ, Melief CJ, Ossendorp F. Abrogation of CTL epitope processing by single amino acid substitution flanking the C-terminal proteasome cleavage site. J Immunol. 164:1898-1905 (2000)). This is assumed to be a cause of persistent infection due to the escape of virus-infected cells from the attack of CTLs in chronic viral diseases such as HIV and HCV (Furutsuki T, Hosoya N, Kawana-Tachikawa A, Tomizawa M, Odawara T, Goto M, Kitamura Y, Nakamura T, Kelleher AD, Cooper DA, Iwamoto A. Frequent transmission of cytotoxic-T-lymphocyte escape mutants of human immunodeficiency virus type 1 in the highly HLA-A24-positive Japanese population. J Virol. 78:8437-8445 (2004)).

Homologies were investigated not only for the epitope region but also for amino acids immediately after the anchor motif at the C terminus of the six types of epitope peptides identified in the present invention.

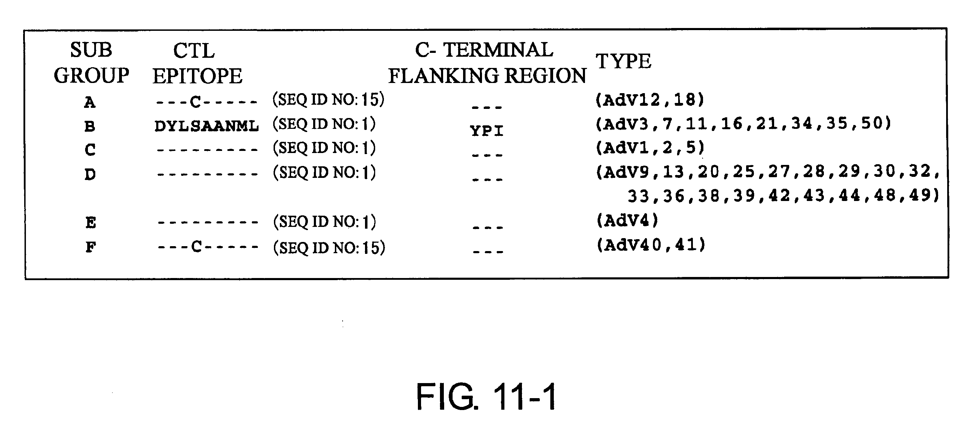

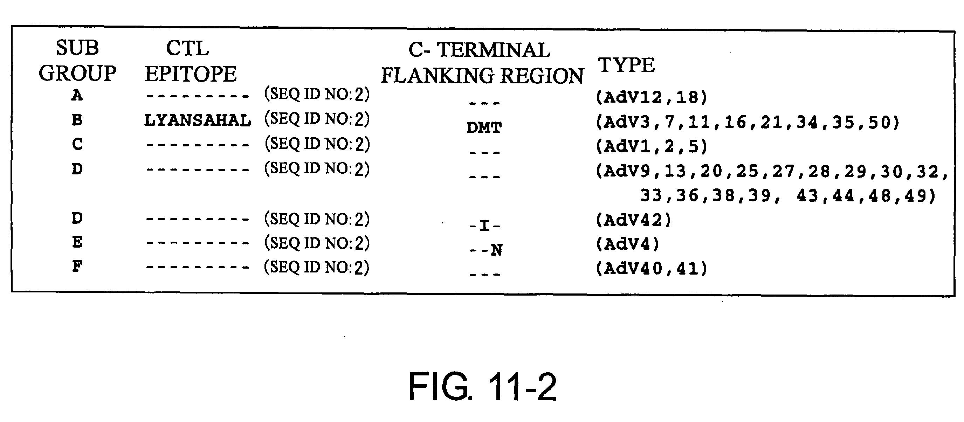

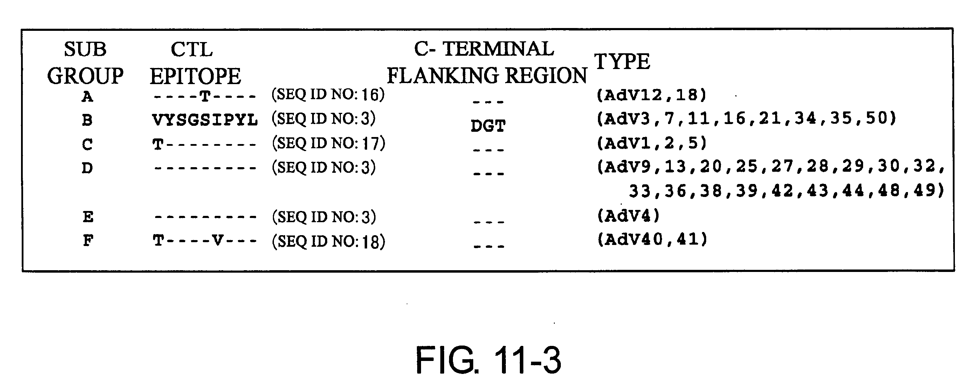

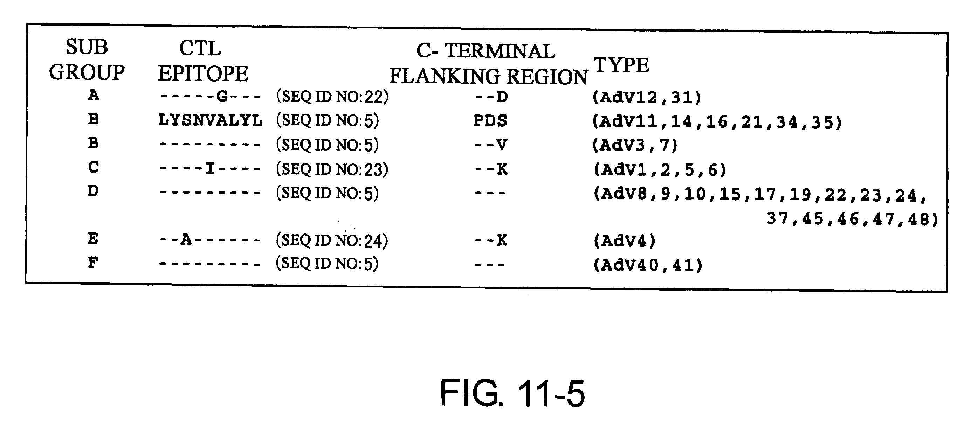

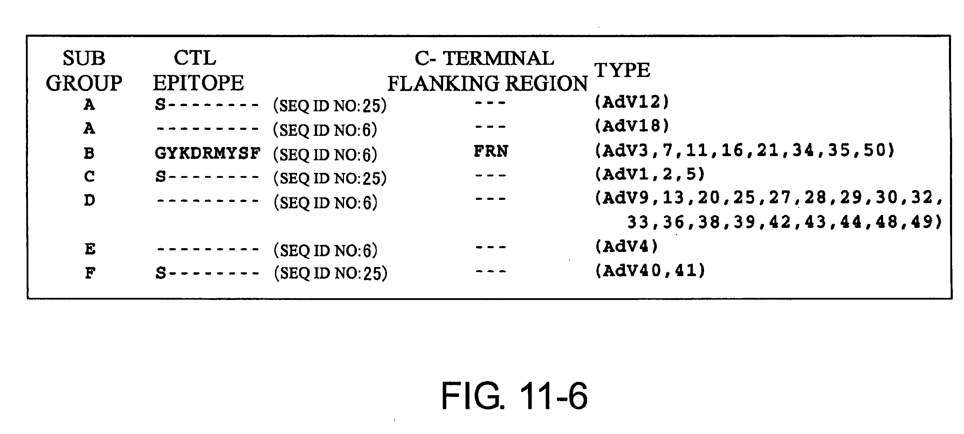

As a result, SEQ ID NO: 1 (DYL) was demonstrated to exhibit 100% homology to adenoviruses belonging to subgroup B, C, D, or E (Fig. 11-1). SEQ ID NO: 2 (LYA) exhibited 100% homology to adenoviruses belonging to any subgroups (Fig. 11-2). SEQ ID NO: 3 (VYS) exhibited 100% homology to adenoviruses belonging to subgroup B, D, or E (Fig. 11-3). SEQ II7 NO: 4 (TYF) exhibited 100% homology only to types 1 l, 21, 34, and 35 adenoviruses belonging to subgroup B (Fig. 11-4). SEQ ID NO: 5 (LYS) exhibited 100% homology to adenoviruses belonging to subgroup B, D, or F (Fig. 11-5). SEQ ID NO: 6 (GYK) exhibited 100% homology to type 18 adenovirus belonging to subgroup A, and adenoviruses belonging to subgroup D or E (Fig. 11-6). In any epitope peptides, no mutation was found in the amino acids immediately after its C terminus. The results are summarized in Table 4. SEQ ID NO: 4 (TYF) was assumed to be a CTL epitope peptide that is highly specific to subgroup B. Other epitope peptides were assumed to be CTL epitope peptides that exhibit a broad specificity not only to subgroup B but also to other subgroups.

This suggests that diagnostic information on the immunity against various types of adenoviruses can be provided using a CTL epitope peptide of the present invention alone or in combination. Furthermore, the epitope peptides were demonstrated to be effective in adoptive immunotherapy, because the peptides can induce adenovirus-specific CTLs that exhibit broad reactivity without adenovirus infection.

**[Table 3]**

| VIRAL SEROTYPE | SUBGROUP | ACCESSION NUMBER | NUMBER OF AMINO ACIDS (aa) | COMMENT |
|---|---|---|---|---|
| 1 | C | AP000512 | 964 | |
| 2 | C | AP000175 | 968 | |
| 3 | B | AAO24104 | 944 | FULL |
| 4 | E | AAS16286 | 936 | |
| 5 | C | AP000211 | 952 | |
| 6 | C | Q04966 | 465 | PARTIAL |
| 7 | B | AP000548 | 934 | |
| 8 | D | P36852 | 517 | PARTIAL |
| 9 | D | CAI05969 | 953 | |
| 10 | D | BAA84982 | 383 | PARTIAL |
| 11 | B | AP000452 | 948 | FULL |
| 12 | A | AP000121 | 919 | |
| 13 | D | BAD15125 | 305 | PARTIAL |
| 14 | B | BAA76594 | 425 | PARTIAL |
| 15 | D | S37277 | 509 | PARTIAL |
| 16 | B | S37216 | 940 | FULL |
| 17 | D | BAA84983 | 378 | PARTIAL |
| 18 | A | CAA76709 | 318 | PARTIAL |
| 19 | D | BAA84984 | 519 | PARTIAL |
| 20 | D | BAD15129 | 305 | PARTIAL |
| 21 | B | AAG21823 | 949 | FULL |
| 22 | D | BAA84985 | 399 | PARTIAL |
| 23 | D | BAA84986 | 404 | PARTIAL |
| 24 | D | BAA94987 | 363 | PARTIAL |
| 25 | D | BAD15133 | 305 | PARTIAL |
| 26 | D | BAA84988 | 376 | PARTIAL |
| 27 | D | BAD 15135 | 305 | PARTIAL |
| 28 | D | BAD 15136 | 305 | PARTIAL |
| 29 | D | BAD 15137 | 305 | PARTIAL |
| 30 | D | BAD 15138 | 305 | PARTIAL |
| 31 | A | P36855 | 468 | PARTIAL |
| 32 | D | BAD15139 | 305 | PARTIAL |
| 33 | D | BAD 15140 | 305 | PARTIAL |
| 34 | B | BAB20014 | 951 | FULL |
| 35 | B | AP 000585 | 952 | FULL |
| 36 | D | BAD 15141 | 305 | PARTIAL |
| 37 | D | BAA84989 | 507 | PARTIAL |
| 38 | D | BAD 15143 | 305 | PARTIAL |
| 39 | D | BAD 15144 | 305 | PARTIAL |
| 40 | F | P11819 | 923 | FULL |
| 41 | F | P11820 | 925 | FULL |
| 42 | D | BAD 15145 | 305 | PARTIAL |
| 43 | D | BAD 15146 | 305 | PARTIAL |
| 44 | D | BAD 15147 | 305 | PARTIAL |
| 45 | D | BAA84990 | 376 | PARTIAL |
| 46 | D | BAA84991 | 364 | PARTIAL |
| 47 | D | BAA84992 | 373 | PARTIAL |
| 48 | D | AAB17439 | 947 | PARTIAL |
| 49 | D | BAD 15152 | 305 | PARTIAL |
| 50 | B | BAD 15153 | 305 | PARTIAL |
| 51 | D | NOT DEPOSITED | | |

**[Table 4]**

| | | SUBGROUP | | | | | |
|---|---|---|---|---|---|---|---|
| | PEPTIDE SEQUENCE | A | B | C | D | E | F |
| SEQ ID NO:1 | DYLSAANML | | ○ | ○ | ○ | ○ | |
| SEQ ID NO:2 | LYANSAHAL | ○ | ○ | ○ | ○ | ○ | ○ |
| SEQ ID NO:3 | VYSGSIPYL | | ○ | | ○ | ○ | |
| SEQ ID NO:4 | TYFNLGNKF | | ○ | | | | |
| SEQ ID NO:5 | LYSNVALYL | | ○ | | ○ | | ○ |
| SEQ ID NO:6 | GYKDRMYSF | ○ | ○ | | ○ | ○ | |

### [Example 12] Vaccine injections

Each of the peptides of SEQ ID NOs: 1 to 6 was dissolved in DMSO at a final concentration being 20 mg/ml. The resulting peptide-containing solutions were sterilized by filtration, and aliquoted into sterile vials at 1 ml/vial. The vials were sealed and provided as vaccine injections.

### Industrial Applicability

When CTLs recognize virus-infected cells, the following characteristics can be noted:
(1) CTLs cannot directly recognize viral particles themselves;
(2) CTLs recognize peptides composed of 8 to 10 amino acids (hereinafter referred to as epitope peptides) within a viral protein, and destroy infected cells;
(3) an epitope peptide is presented by binding with the complex of human leukocyte antigen (hereinafter, referred to as HLA) and β2-microglobulin present on the surface of virus-infected cells, and CTLs recognize and destroy virus-infected cells through binding of T cell receptors (TCR) on the surface of CTLs thereto; and
(4) HLAs differ among races and individuals, and when the HLA is different, the epitope peptides are different even for the same virus (this is called "HLA restriction of epitope peptide").

As is apparent from the description of (1) to (4), adenovirus-specific epitope peptides differ depending on the type of HLA, and thus, are restricted by HLA. The present inventors analyzed adenovirus-specific CTL epitope peptides for, for example, the HLA-A24 type, which is frequently found worldwide and carried by about 60% of Japanese people, and as a result succeeded in providing epitope peptides useful in diagnosing whether effective immunity against adenovirus infection is present, as well as in treating or preventing adenovirus infection.

In the context of the present invention, the term "CTLs" refers to cytotoxic T cells that express CD8 or CD4, one of surface antigen molecules present on human T cells, and that are restricted by HLA class I. Furthermore, adenovirus-specific CTLs of the present invention refer to T cells that can eliminate adenovirus-infected cells by directly damaging the adenovirus-infected cells when T cell receptor (hereinafter abbreviated as TCR) expressed on the membrane surface of CTLs recognizes the ternary complex of adenovirus-specific HLA class I-restricted epitope peptide, and HLA class I molecule and β2-microglobulin expressed on the cell membrane surface of adenovirus-infected cells. Alternatively, the adenovirus-specific HLA class I-restricted epitope peptide refers to a peptide composed of a sequence of 8 to 10 amino acids at a particular position in an adenovirus protein that can be recognized by CTLs and form a ternary complex with HLA class I molecule and β2-microglobulin, which is a structural moiety of adenovirus to which TCR on the membrane surface of CTLs can immunologically recognize and bind.

When used as vaccines, the epitope peptides of the present invention can induce adenovirus-specific CTLs in the body, to thereby retain immunity against adenovirus infection. The peptides can artificially induce and grow adenovirus-specific CTLs *ex vivo* in a safe and efficient way without adenovirus infection of biological samples, such as peripheral blood. Thus, the peptides find utility in the context of cellular immunotherapy. Furthermore, since the peptides can be used to diagnose the presence of immunity against adenovirus, they serve as effective therapeutic and diagnostic methods for adenovirus infection.

## Claims

1. An adenovirus-specific cytotoxic T cell epitope peptide.

2. The peptide of claim 1, wherein the adenovirus-specific cytotoxic T cell epitope peptide comprises at least one amino acid sequence selected from the group consisting of SEQ ID NOs: 1 to 6.

3. The peptide of claim 1 comprising an amino acid sequence with a substitution, deletion, insertion, and/or addition of one or more amino acids in the amino acid sequence of any one of SEQ ID NOs: 1 to 6, which has the function capable of inducing an adenovirus-specific cytotoxic T cell.

4. The peptide of any one of claims 1 to 3, wherein the peptide comprises an antigen peptide restricted by HLA-A*2402, HLA-Cw*0401, or HLA-Cw*0702 molecule and has the function capable of inducing a cytotoxic T cell having a T cell receptor capable of specifically recognizing a cell that presents a complex with HLA-A*2402, HLA-Cw*0401, or HLA-Cw*0702 molecule on the cell surface.

5. A nucleic acid encoding the peptide of any one of claims 1 to 4.

6. A vaccine for treating or preventing adenovirus infection, which comprises as an active ingredient the peptide of any one of claims 1 to 4,.

7. A vaccine for treating or preventing adenovirus infection, which comprises as an active ingredient the nucleic acid of claim 5.

8. A vaccine for treating or preventing adenovirus infection, which comprises as an active ingredient an antigen-presenting cell that presents the peptide of any one of claims 1 to 4 by HLA.

9. A passive immunotherapeutic agent against adenovirus, which comprises as an active ingredient an adenovirus-specific cytotoxic T cell obtained by stimulating a peripheral blood lymphocyte with the peptide of any one of claims 1 to 4 or an antigen-presenting cell that presents said peptide by HLA.

10. A passive immunotherapeutic agent against adenovirus, which comprises as an active ingredient a cytotoxic T cell that is obtained by reacting a peripheral blood lymphocyte with a major histocompatibility antigen complex and/or major histocompatibility antigen complex-tetramer prepared from the peptide of any one of claims 1 to 4, allowing the formation of a complex in which said major histocompatibility antigen complex and/or major histocompatibility antigen complex-tetramer are bound with a cytotoxic T cell, and isolating the cytotoxic T cell from said complex.

11. A method for quantifying adenovirus-specific cytotoxic T cells, which comprises: stimulating peripheral blood with the peptide of any one of claims 1 to 4, obtaining cytotoxic T cells specific to said virus, and assaying a cytokine and/or chemokine and/or cell surface molecule produced by the cytotoxic T cells.

12. A method for quantifying adenovirus-specific cytotoxic T cells in peripheral blood, which comprises: preparing a major histocompatibility antigen complex-tetramer from the peptide of any one of claims 1 to 4, and reacting the peripheral blood with the major histocompatibility antigen complex-tetramer.

13. A method for inducing a cytotoxic T cell, which comprises inducing a cytotoxic T cell using the peptide of any one of claims 1 to 4.

14. The method of claim 13, wherein an adenovirus-specific cytotoxic T cell is induced by contacting the peptide of any one of claims 1 to 4 with a peripheral blood mononuclear cell in a culture medium containing plasma.

15. A method for producing a passive immunotherapeutic agent against adenovirus, which comprises the step of obtaining an adenovirus-specific cytotoxic T cell by stimulating a peripheral blood lymphocyte with the peptide of any one of claims 1 to 4 or an antigen-presenting cell that presents said peptide by HLA.

16. A method for producing a passive immunotherapeutic agent against adenovirus, which comprises the step of obtaining a cytotoxic T cell by reacting a peripheral blood lymphocyte with a major histocompatibility antigen complex and/or major histocompatibility antigen complex-tetramer prepared from the peptide of any one of claims 1 to 4, allowing the formation of a complex in which said major histocompatibility antigen complex and/or major histocompatibility antigen complex-tetramer are bound with the cytotoxic T cell, and isolating the cytotoxic T cell from said complex.
